# EUROPEAN PATENT APPLICATION

(11) **EP 2 039 680 A1**
(43) Date of publication of application: **25.03.2009**
(21) Application number: 07790171.8
(22) Date of filing: 19.06.2007
(51) Int. Cl.: C07C 251/30, C07C 57/10, C07C 249/02, C07C 303/28, C07C 303/32, C07C 309/04, C07C 309/73, C07D 207/12, C07D 207/16, C07D 233/06

(54) **PROCESS FOR PRODUCING ESTER OR ALCOHOL**

(30) Priority: 20.06.2006 JP 2006170546
(71) Applicant: Mitsui Chemicals, Inc., Tokyo 105-7117 (JP)
(72) Inventor: TSUNODA, Hidetoshi, Omuta-shi, Fukuoka 836-8610 (JP); FUKUMURA, Kouki, Omuta-shi, Fukuoka 836-8610 (JP)
(74) Representative: Stuart, Ian Alexander
(86) International application number: PCT/JP2007/000645
(87) International publication number: WO 2007/148435

(57) **Abstract**

Provided is a process for producing an ester or alcohol using a fluoroimidinium sulfonate derivative represented by the general formula (9) or a fluoroimidinium carboxylate derivative represented by the general formula (6) and using as a raw material alcohol involving inversion of steric configuration. Further provided are a fluoroimidinium sulfonate derivative represented by the general formula (9), and a process for producing the same.

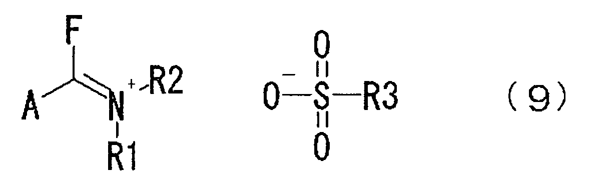

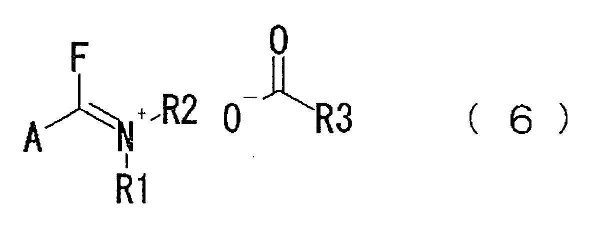

## Description

### TECHNICAL FIELD

The present invention relates to a novel fluorine-containing compound, and to a process for producing an ester or alcohol having an inverted steric configuration with respect to alcohol serving as a raw material. More specifically, the invention relates to a fluoroimidinium sulfonate derivative that is a fluorine-containing compound, to a process for producing the same, and to a process for producing a sulfonic acid ester using the same with the use of alcohol as a raw material. Further, the invention relates to a fluoroimidinium carboxylate derivative that is a fluorine-containing compound, to a process for producing the same, and to a process using the same, which is for producing a carboxylic acid ester or alcohol derivative having an inverted steric configuration with respect to alcohol serving as a raw material. The ester and the alcohol produced by the invention allow a variety of desired functional groups to be easily introduced, and have configurations requiring high optical purity. Therefore, they belong to a group of the compounds that are useful as raw materials for preparation in a wide field including raw materials for pharmaceuticals, agrochemicals, cosmetics and the like.

### BACKGROUND ART

As for a process for producing a sulfonic acid ester derivative using alcohol as a raw material, the related art is described with reference to the following processes (1) through (3).

### (1) Process in which a sulfonic acid halide is used

This is a process in which alcohol is reacted with a sulfonic acid halide to produce a corresponding sulfonic acid ester derivative. In most cases, the halide is chloride or fluoride (Non-Patent Document 1). Generally, the reaction cannot be completed due to an effect of an acid component that is a side product, unless it is carried out in the coexistence of a base of at least molar equivalence. Furthermore, there is also a process including the reaction with a metal alkoxide that has been separately prepared (Non-Patent Document 2). As the base, pyridine, triethylamine, or the like is used in many cases. However, there are problems, such as that the resulting sulfonic acid ester derivative decomposes by an effect of the coexisting base, that an optically active compound is racemized which thereby lowering the optical purity, or the like. If an optically active alcohol is used as a raw material, a corresponding sulfonic acid ester derivative maintaining its configuration can be obtained in the case of not undergoing a racemization.

### (2) Process in which a sulfonic acid anhydride is used

This is a process in which alcohol is reacted with a sulfonic acid anhydride to produce a corresponding sulfonic acid ester derivative (Non-Patent Document 3). The reaction is not completed unless a base coexists. Problems are that, as in (1) above, the resulting sulfonic acid ester derivative decomposes by an effect of the coexisting base, that an optically active compound is racemized which thereby lowering the optical purity, or the like. If an optically active alcohol is used as a raw material, a corresponding sulfonic acid ester derivative maintaining its configuration can be obtained in the case of not undergoing a racemization.

### (3) Process by a Mitsunobu reaction

This is a process in which alcohol is reacted with a sulfonic acid in the coexistence of an azodicarboxylic acid ester and a phosphine derivative to produce a corresponding sulfonic acid ester derivative (Non-Patent Documents 4 and 5). When optically active alcohol is used as a raw material, it is characterized by involving inversion of the configuration, unlike the above-described (1) and (2), whereby a sulfonic acid ester derivative can be obtained. The Mitsunobu reaction has a problem in that it is difficult to separate a sulfonic acid ester derivative, which is the desired product, from an azodicarboxylic acid ester and a compound derived from a phosphine derivative which are side products. In addition, the azodicarboxylic acid ester to be used has a risk of explosion, and further, it is not suitable for the reaction performed in an industrial scale from the viewpoint of its relatively high cost.

As for the process for producing a carboxylic acid ester or alcohol derivative having an inverted steric configuration using alcohol as a raw material, the related art is described with reference to the following (4).

### (4) Process by a Mitsunobu reaction

This is a process in which alcohol is reacted with a carboxylic acid in the coexistence of an azodicarboxylic acid ester and a phosphine derivative to produce a corresponding carboxylic acid ester derivative (Non-Patent Document 6). When optically active alcohol is used as a raw material, inversion of the configuration is involved, whereby a carboxylic acid ester derivative can be obtained. The Mitsunobu reaction has a problem in that it is difficult to separate a carboxylic acid ester derivative, which is the desired product, from an azodicarboxylic acid ester and a compound derived from a phosphine derivative which are side products. In addition, the azodicarboxylic acid ester to be used has a risk of explosion, and further, it is not suitable for the reaction performed in an industrial scale from the viewpoint of its relatively high cost.

In addition, it is generally known that the obtained carboxylic acid ester derivative can be subject to a hydrolysis reaction under a basic or acidic condition, or to a reductive reaction using lithium aluminum hydride, sodium borohydride, or the like so as to produce a corresponding alcohol derivative.

Furthermore, tetraalkylfluoroamidinium trifluoroacetate that is within the scope of the fluoroimidinium carboxylate derivative to be used in the invention is well-known as a trifluoroacetoxylating agent. However, it has not been known at all that a carboxylic acid ester or alcohol involving inversion of steric configuration can be prepared from alcohol with a high stereoselectivity using a fluoroimidinium carboxylate derivative, as in the invention (Patent Document 1).
[Non-Patent Document 1] R. Stuart Tipson, J. Org. Chem., pp. 235 to 241 (1944)
[Non-Patent Document 2] Herbert C. Brown, et al., J. Am. Chem. Soc., 89, pp. 370 to 378 (1967)
[Non-Patent Document 3] L. R. Subramanian, et al., Chem. Ber. , 105, pp. 1465 to 1470 (1972)
[Non-Patent Document 4] Igor Galynker, et al., Tetrahedron Lett., 23, pp. 4461 to 4464 (1982)
[Non-Patent Document 5] Neal G. Anderson, et al., J. Org. Chem. , 61, pp. 7955 to 7958 (1996)
[Non-Patent Document 6] Oyo Mitsunobu, Synthesis, pp. 1 to 28 (1981)
[Patent Document 1] Japanese Unexamined Patent Application Publication No. 2000-128868

### DISCLOSURE OF THE INVENTION

The present invention provides (1) a novel process for producing an ester or alcohol using as a raw material alcohol having an optical activity or geometric isomerism, and involving inversion of steric configuration with respect to alcohol serving as a raw material, which is suitable for industrial production. In addition, the invention provides (2) a fluoroimidinium sulfonate derivative and fluoroimidinium carboxylate derivative, which are each a very useful reaction reagent for the preparation of a sulfonic acid ester, a carboxylic acid ester and alcohol using alcohol as a raw material, and a process for producing the same.
According to the invention, the following are provided.
[1] A process for producing an ester or alcohol having an inverted steric configuration with respect to alcohol serving as a raw material, the process includes reacting an optically active alcohol or a geometrically isomeric alcohol with a compound represented by the general formula (1): 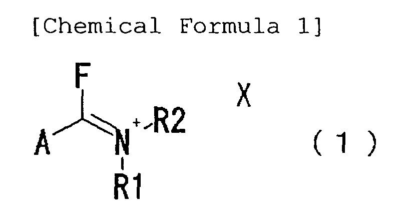 [wherein R1 and R2 each independently represents a substituted or unsubstituted alkyl group, a substituted or unsubstituted aryl group, or a substituted or unsubstituted hetero ring, and may be the same as or different from each other. Further, R1 and R2 may be bonded to each other to form a ring containing a nitrogen atom, or a nitrogen atom and other heteroatom. A represents a hydrogen atom, a substituent represented by the general formula (2) 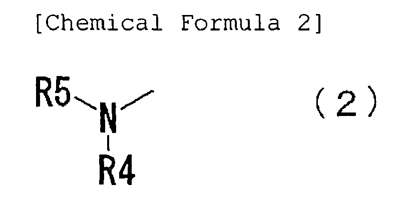 (wherein R4 and R5 each independently represents a substituted or unsubstituted alkyl group, a substituted or unsubstituted aryl group, or a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.), or a substituent represented by the general formula (3) 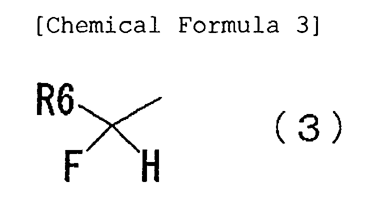 (wherein R6 represents a fluorine atom, or an alkyl group that is partially or fully substituted with fluorine atom(s)). X represents a compound represented by the general formula (4) 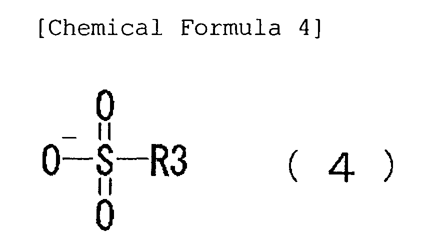 (wherein R3 represents a substituted or unsubstituted alkyl group, a substituted or unsubstituted aryl group, or a substituted or unsubstituted hetero ring), or by the general formula (5) 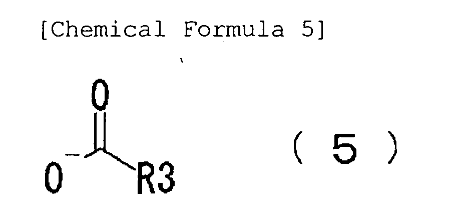 (wherein R3 represents a substituted or unsubstituted alkyl group, a substituted or unsubstituted aryl group, or a substituted or unsubstituted hetero ring)].
[2] The process for producing an ester or alcohol as described in [1], wherein in the compound represented by the general formula (1), X is represented by the general formula (4), and the process includes obtaining a sulfonic acid ester.
[3] The process for producing an ester or alcohol as described in [1], wherein the compound represented by the general formula (1) is represented by the general formula (6) 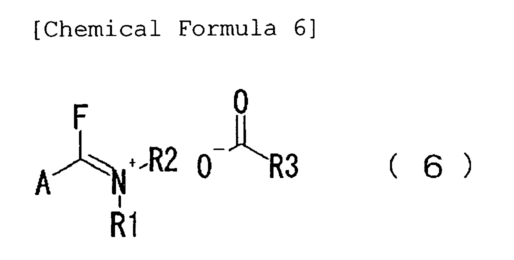 (wherein R1, R2, R3 and A each have the same meanings as defined in the general formula (1)), and the process includes obtaining a carboxylic acid ester or alcohol.
[4] The process for producing an ester or alcohol as described in [3], wherein the compound represented by the general formula (6) is represented by the general formula (7) 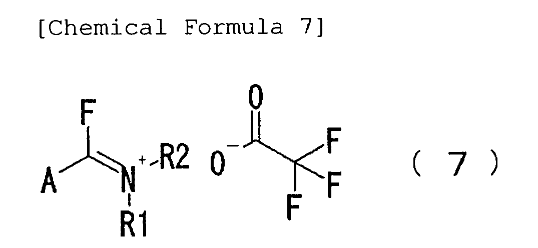 (wherein R1, R2 and A each have the same meanings as defined in the general formula (1)), and the process includes obtaining a carboxylic acid ester or alcohol.
[5] The process for producing an ester or alcohol as described in [2], wherein the optically active alcohol or the geometrically isomeric alcohol is an optically active secondary alcohol, and the process includes obtaining a sulfonic acid ester.
[6] The process for producing an ester or alcohol as described in [3] or [4], wherein the optically active alcohol or the geometrically isomeric alcohol is an optically active secondary alcohol, and the process includes obtaining a carboxylic acid ester or alcohol.
[7] The process for producing an ester or alcohol as described in [5], wherein the optically active secondary alcohol is represented by the general formula (8) 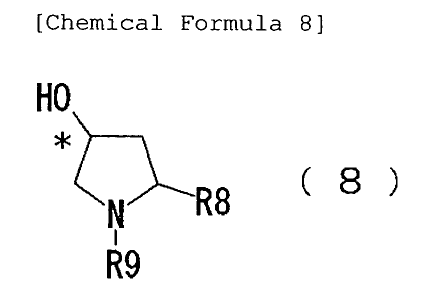 (wherein R8 represents a hydrogen atom, a carboxylic acid, a carboxylic acid ester, or a carboxylic acid amide. R9 represents a substituted or unsubstituted alkyl group, a substituted or unsubstituted aryl group, a substituted or unsubstituted alkoxycarbonyl group, a substituted or unsubstituted aryloxycarbonyl group, a substituted or unsubstituted alkylcarbonyl group, or a substituted or unsubstituted arylcarbonyl group. The symbol * represents that the hydroxyl group is optically active, indicating a compound in either an R- or an S-form.), and the process includes obtaining a sulfonic acid ester.
[8] The process for producing an ester or alcohol as described in [6], wherein the optically active secondary alcohol is represented by the general formula (8) 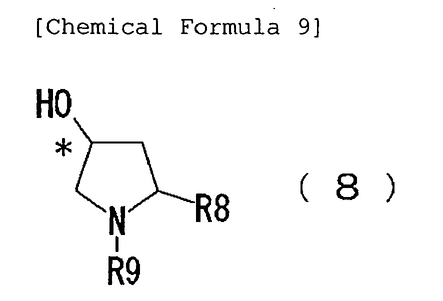 (wherein R8 represents a hydrogen atom, a carboxylic acid, a carboxylic acid ester, or a carboxylic acid amide. R9 represents a substituted or unsubstituted alkyl group, a substituted or unsubstituted aryl group, a substituted or unsubstituted alkoxycarbonyl group, a substituted or unsubstituted aryloxycarbonyl group, a substituted or unsubstituted alkylcarbonyl group, or a substituted or unsubstituted arylcarbonyl group. The symbol * represents that the hydroxyl group is optically active, indicating a compound in either an R- or an S-form.), and the process includes obtaining a carboxylic acid ester or alcohol.
[9] A compound represented by the general formula (9) 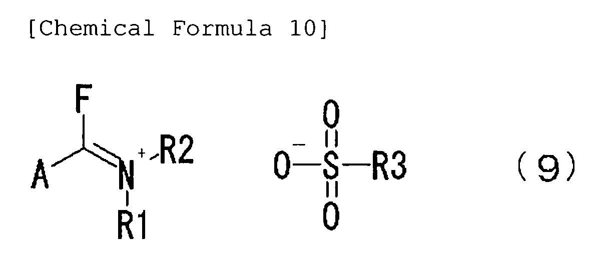 [wherein R1, R2, and R3 each independently represents a substituted or unsubstituted alkyl group, a substituted or unsubstituted aryl group, or a substituted or unsubstituted hetero ring, and may be the same as or different from each other. Further, R1 and R2 may be bonded to each other to form a ring containing a nitrogen atom, or a nitrogen atom and other heteroatom. A represents a hydrogen atom, a substituent represented by the general formula (2) 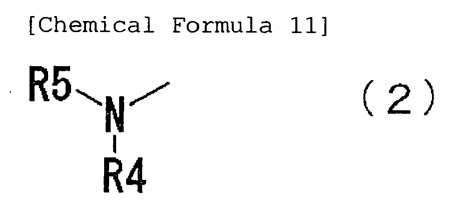 (wherein R4 and R5 each independently represents a substituted or unsubstituted alkyl group, a substituted or unsubstituted aryl group, a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.), or a substituent represented by the general formula (3): 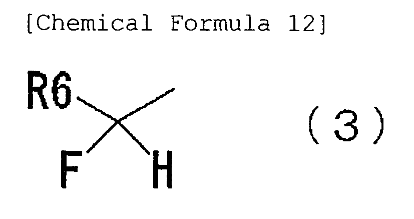 (wherein R6 represents a fluorine atom, or an alkyl group that is partially or fully substituted with fluorine atom(s))].
[10] The compound as described in [9], wherein the compound represented by the general formula (9) is represented by the general formula (10) 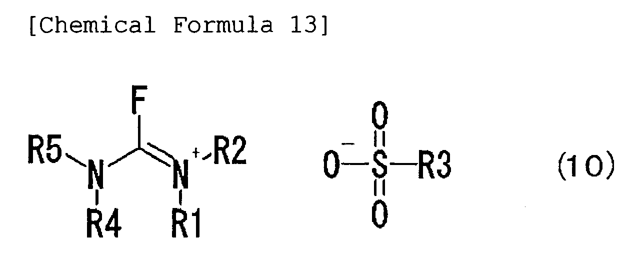 (wherein R1 to R5 each have the same meanings as defined in the general formula (9)).
[11] The compound as described in [9], wherein the compound represented by the general formula (9) is represented by the general formula (11) 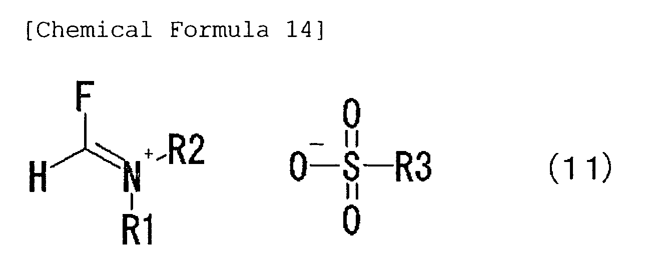 (wherein R1 to R3 each have the same meanings as defined in the general formula (9)).
[12] The compound as described in [9], wherein the compound represented by the general formula (9) is represented by the general formula (12) 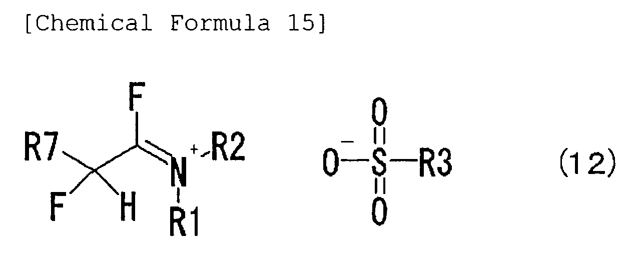 (wherein R1 to R3 each have the same meanings as defined in the general formula (9). R7 represents a fluorine atom, or an alkyl group having 1 to 6 carbon atoms, that is fully substituted with fluorine atoms).
[13] The compound as described in [10], wherein in the compound represented by the general formula (10), R1, R2, R4, and R5 are each independently an alkyl group having 1 to 6 carbon atoms.
[14] The compound as described in [10], wherein the compound represented by the general formula (10) is represented by the general formula (13) 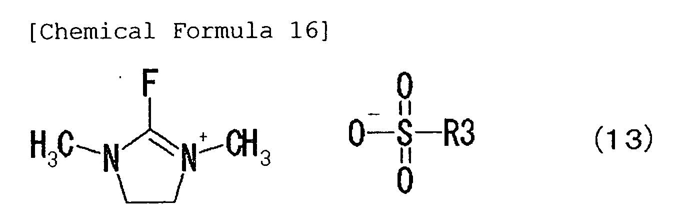 (wherein R3 has the same meaning as defined in the general formula (9)).
[15] A process for producing a compound represented by the general formula (9): 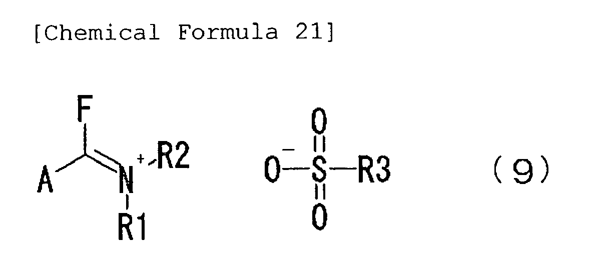 [wherein R1, R2, R3, and A each has the same meanings as defined below],
   the process including reacting a compound represented by the general formula (14): 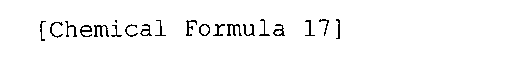 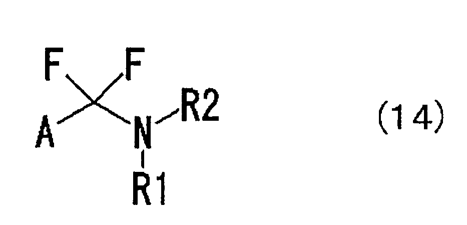 [wherein R1 and R2 each independently represents a substituted or unsubstituted alkyl group, a substituted or unsubstituted aryl group, or a substituted or unsubstituted hetero ring, and may be the same as or different from each other, where R1 and R2 may be bonded to each other to form a ring containing a nitrogen atom, or a nitrogen atom and other heteroatom; A represents a hydrogen atom, a substituent represented by the general formula (2): 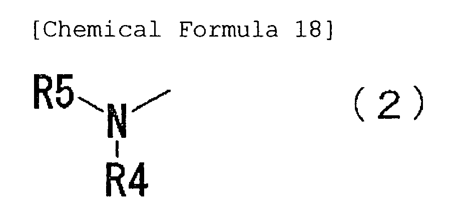 (wherein R4 and R5 each independently represents a substituted or unsubstituted alkyl group, a substituted or unsubstituted aryl group, or a substituted or unsubstituted hetero ring, and may be the same as or different from each other, where R4 and R5 may be bonded to each other to form a ring containing a nitrogen atom, or a nitrogen atom and other heteroatom; alternatively, R1 and R4 may be bonded to each other to form a ring containing a nitrogen atom.), or a substituent represented by the general formula (3): 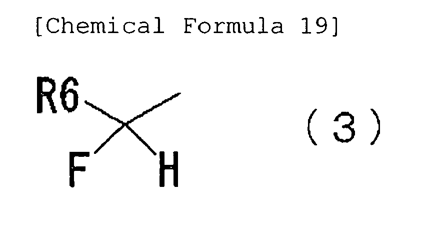 (wherein R6 represents a fluorine atom, or an alkyl group that is partially or fully substituted with fluorine atom(s)); any one of the fluorine atoms on the carbon atoms bonded with the nitrogen atoms may be anions to form ion-pairs] with a compound represented by the general formula (15): 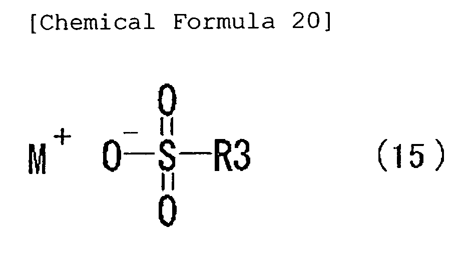 (wherein R3 represents a substituted or unsubstituted alkyl group, a substituted or unsubstituted aryl group, or a substituted or unsubstituted hetero ring; M⁺ represents an alkali metal ion or an alkaline earth metal ion.).
[16] The process as described in [15], wherein the compound represented by the general formula (14) is represented by the general formula (16) 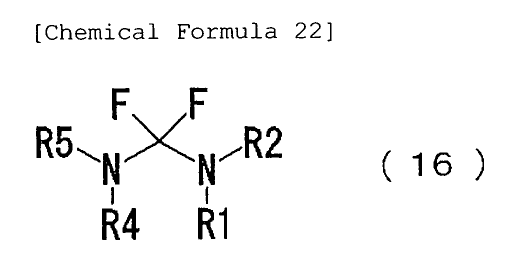 (wherein R1, R2, R4, and R5 each have the same meanings as defined in the general formula (14). Further, any one of the fluorine atoms on the carbon atoms bonded with the nitrogen atoms may be anions to form ion-pairs).
[17] The process as described in [15], wherein the compound represented by the general formula (14) is represented by the general formula (17) 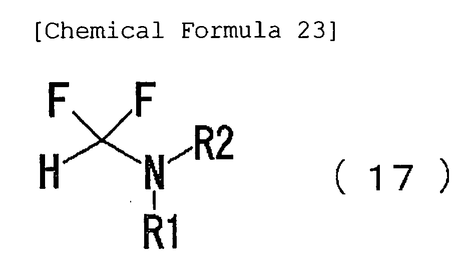 (wherein R1 and R2 each have the same meanings as defined in the general formula (14). Further, any one of the fluorine atoms on the carbon atoms bonded with the nitrogen atoms may be anions to form ion-pairs.).
[18] The process as described in [15], wherein the compound represented by the general formula (14) is represented by the general formula (18) 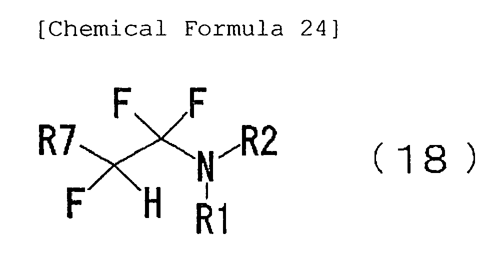 (wherein R1 and R2 each have the same meanings as defined in the general formula (14). R7 represents a fluorine atom, or an alkyl group having 1 to 6 carbon atoms, that is fully substituted with fluorine atoms. Further, any one of the fluorine atoms on the carbon atoms bonded with the nitrogen atoms may be anions to form ion-pairs. Alternatively, the hydrogen fluoride may leave to make an olefinic compound.).
[19] The process as described in [15], wherein the compound represented by the general formula (14) is the compound in which R1, R2, R4, and R5 are each an alkyl group having 1 to 6 carbon atoms.
[20] The process as described in [15], wherein the compound represented by the general formula (14) is represented by the formula (19) 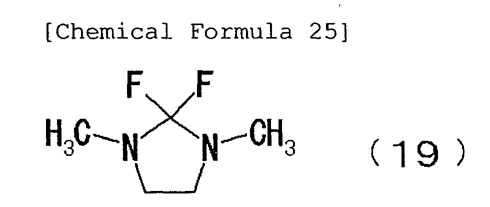
[21] A process for producing a sulfonic acid ester, including reacting alcohol with a compound represented by the general formula (9) 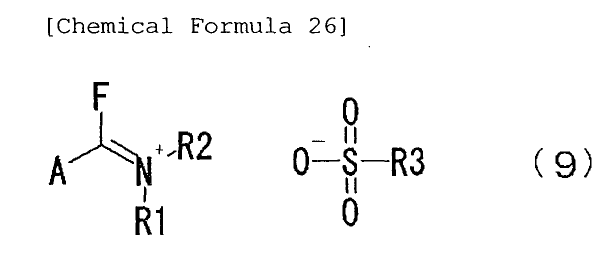 [wherein R1, R2, and R3 each independently represents a substituted or unsubstituted alkyl group, a substituted or unsubstituted aryl group, a substituted or unsubstituted hetero ring, and may be the same as or different from each other. Further, R1 and R2 may be bonded to each other to form a ring containing a nitrogen atom, or a nitrogen atom and other heteroatom. A represents a hydrogen atom, a substituent represented by the general formula (2) 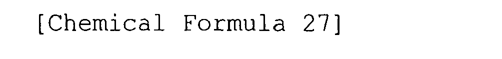 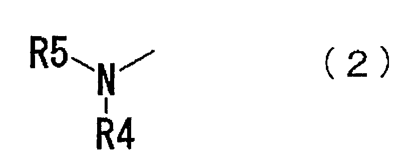 (wherein R4 and R5 each independently represents a substituted or unsubstituted alkyl group, a substituted or unsubstituted aryl group, or a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.), or a substituent represented by the general formula (3) 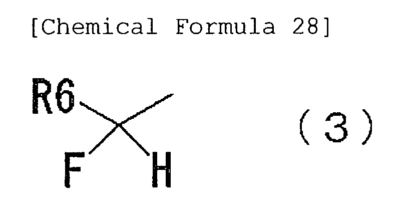 (wherein R6 represents a fluorine atom, or an alkyl group that is partially or fully substituted with fluorine atom(s).).

According to the invention, a sulfonic acid ester, a carboxylic acid ester and alcohol using alcohol as a raw material can be prepared in a simple manner and with a high yield. Further, a sulfonic acid ester, a carboxylic acid ester and alcohol can be derived by involving inversion of steric configuration with respect to alcohol serving as a raw material. In addition, the by-products derived from the reaction reagent are easily removed, and there is no risk of explosion, thus providing high stability. Therefore, a very efficient preparation process from an industrial viewpoint is provided.

### BEST MODE FOR CARRYING OUT THE INVENTION

Hereinbelow, the present invention is described in detail.
The general formula (1) is represented as below. 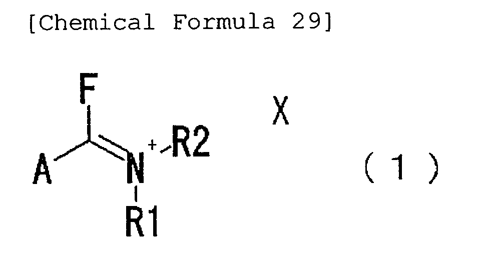 (wherein R1 and R2 each independently represents a substituted or unsubstituted alkyl group, a substituted or unsubstituted aryl group, or a substituted or unsubstituted hetero ring, and may be the same as or different from each other. Further, R1 and R2 may be bonded to each other to form a ring containing a nitrogen atom, or a nitrogen atom and other heteroatom).

In the general formula (1), the "unsubstituted or substituted alkyl group" in R1 and R2 means an alkyl group in which any hydrogen atom is substituted with a substituent, or an unsubstituted alkyl group. Examples of the alkyl group include a methyl group, an ethyl group, an isopropyl group, an n-butyl group, a tert-butyl group, an n-pentyl group, an n-hexyl group, a cyclohexyl group, an octyl group, a decyl group, a hexadecyl group, a vinyl group, and an allyl group. Further, examples of the substituent of the alkyl group include an alkyl group such as a methyl group, an ethyl group, an isopropyl group, an n-butyl group, a tert-butyl group, an n-pentyl group, an n-hexyl group, a cyclohexyl group, an octyl group, a decyl group, a hexadecyl group, a vinyl group, and an allyl group, an alkoxy group such as a methoxy group, a benzyloxy group, and a methoxyethoxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

In the general formula (1), the "unsubstituted or substituted aryl group" in R1 and R2 means an aryl group in which any hydrogen atom is substituted with a substituent, or an unsubstituted aryl group. Examples of the aryl group include a phenyl group, a naphthyl group, a biphenyl group, an anthracenyl group, a pyridyl group, a quinolyl group, a furyl group, and a thienyl group. Further, examples of the substituent of the aryl group include an alkyl group such as a methyl group, an ethyl group, an isopropyl group, an n-butyl group, a tert-butyl group, an n-pentyl group, an n-hexyl group, a cyclohexyl group, an octyl group, a decyl group, a hexadecyl group, a vinyl group, and an allyl group, an alkoxy group such as a methoxy group, a benzyloxy group, and a methoxyethoxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

In the general formula (1), the "substituted or unsubstituted hetero ring" in R1 and R2 means a hetero ring in which any hydrogen atom is substituted with a substituent, or an unsubstituted hetero ring. Examples of the hetero ring include pyrrolidine, piperidine, piperazine, tetrahydrofuran, tetrahydropyran, tetrahydrothiophene, morpholine, and dioxane. Further, examples of the substituent of the hetero ring include an alkyl group such as a methyl group, an ethyl group, an isopropyl group, an n-butyl group, a tert-butyl group, an n-pentyl group, an n-hexyl group, a cyclohexyl group, an octyl group, a decyl group, a hexadecyl group, a vinyl group, and an allyl group, an alkoxy group such as a methoxy group, a benzyloxy group, and a methoxyethoxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

In the general formula (1), the "R1 and R2 may be bonded to each other to form a ring containing a nitrogen atom, or a nitrogen atom and other heteroatom" means that R1 and R2 may be bonded to form a ring containing a nitrogen atom, or to form a ring containing a nitrogen atom and other heteroatom. Examples of the ring including a nitrogen atom include pyrazole, pyrrolidine, imidazole, piperidine, indole, benzimidazole, and carbazole. Examples of the ring including a nitrogen atom and other heteroatom include morpholine and thiomorpholine.

In the general formula (1), A represents a hydrogen atom, a substituent represented by the following general formula (2), or a substituent represented by the following general formula (3).
The general formula (2) is represented as below. 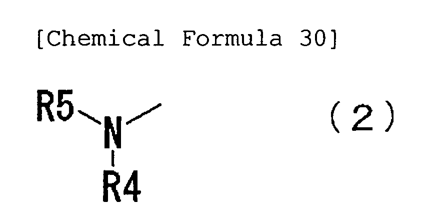 (wherein R4 and R5 each independently represents a substituted or unsubstituted alkyl group, a substituted or unsubstituted aryl group, or a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.)

In the general formula (2), the "substituted or unsubstituted alkyl group", the "substituted or unsubstituted aryl group", the "substituted or unsubstituted hetero ring" each have the same meanings as defined for the "substituted or unsubstituted alkyl group", the "substituted or unsubstituted aryl group", the "substituted or unsubstituted hetero ring" in R1 and R2 in the general formula (1).

In the general formula (2), the "R4 and R5 may be bonded to each other to form a ring containing a nitrogen atom, or a nitrogen atom and other heteroatom" means that R4 and R5 may be bonded to form a ring containing a nitrogen atom, or a ring containing a nitrogen atom and other heteroatom. Examples of the ring including a nitrogen atom include pyrazole, pyrrolidine, imidazole, piperidine, indole, benzimidazole, and carbazole. Examples of the ring including a nitrogen atom and other heteroatom include morpholine and thiomorpholine.

In the general formula (2), R1 and R4 may be bonded to each other to form a ring containing a nitrogen atom. Examples of the ring including a nitrogen atom include imidazoline, tetrahydropyrimidine, and benzimidazoline.

The general formula (3) is represented as below.

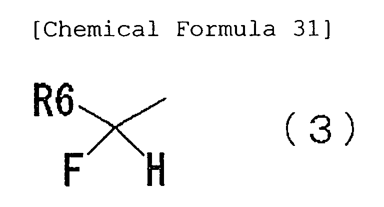

(wherein R6 represents a fluorine atom, or an alkyl group that is partially or fully substituted with fluorine atom(s).)

In the general formula (3), the "alkyl group that is partially or fully substituted with fluorine atom(s)" of R6 means an alkyl group in which a part of the hydrogen atoms is substituted with a fluorine atom, or all of the hydrogen atoms are substituted with fluorine atoms. Examples of the alkyl group include a methyl group, an ethyl group, an isopropyl group, an n-butyl group, a tert-butyl group, an n-pentyl group, an n-hexyl group, a cyclohexyl group, an octyl group, a decyl group, a hexadecyl group, a vinyl group, and an allyl group.

In the general formula (1), X represents a compound represented by the following general formula (4) or the following general formula (5).
The general formula (4) is represented as below.

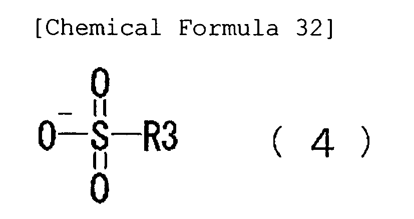

In the formula, R3 represents a substituted or unsubstituted alkyl group, a substituted or unsubstituted aryl group, or a substituted or unsubstituted hetero ring. As mentioned herein, the "substituted or unsubstituted alkyl group", the "substituted or unsubstituted aryl group", and the "substituted or unsubstituted hetero ring" each have the same meanings as described above in R1 and R2.
The general formula (5) is represented as below.

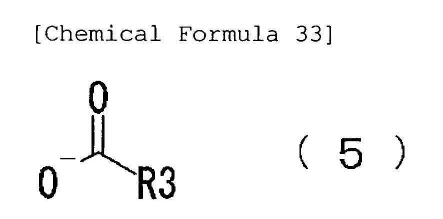

(wherein R3 represents a substituted or unsubstituted alkyl group, a substituted or unsubstituted aryl group, or a substituted or unsubstituted hetero ring).

The invention is based on a very useful finding that if the compound represented by the general formula (1) is reacted with optically active alcohol or geometrically isomeric alcohol as a raw material, an ester or alcohol can be obtained with the involvement of inversion of steric configuration. In conventional esterifying agents, esterification or derivation of alcohol could be attained by involving inversion of steric configuration, only through the use of a Mitsunobu reaction. However, the process using the Mitsunobu reaction had been a preparation process that is not suitable in an industrial scale from the viewpoint of isolation and stability of a desired product, and preparation cost. As compared with the Mitsunobu reaction, this inventive reaction is a preparation process that allows easy isolation and purification due to no use of a phosphine derivative, and is safe since it does not require a diazocarboxylic acid ester having a risk of explosion.

The compound represented by the general formula (1) is represented, for example, by the following general formula (9).

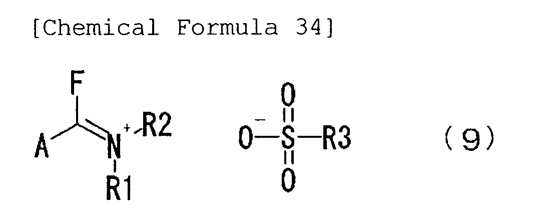

(wherein R1, R2, R3, and A each have the same meanings as defined above).

The compound represented by the general formula (9) has an excellent ability as a sulfonic acid esterifying agent using alcohol as a raw material. That is, it makes it possible to prepare a sulfonic acid ester having an inverted steric configuration with respect to alcohol serving as a raw material. Further, after the sulfonic acid esterification reaction, it is easy to separate a desired compound from the compound derived from the reagent, and the process is highly safe since there is no concern about explosion. Accordingly, it is very useful from an industrial viewpoint.

Further, the compound represented by the general formula (1) is represented by the following general formula (6), for example. 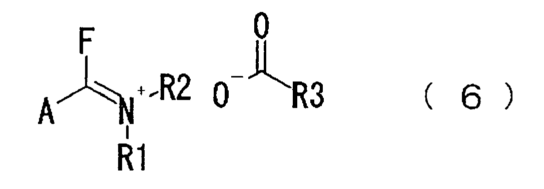 (wherein R1, R2, R3 and A each have the same meanings as defined above.)

The compound represented by the general formula (6) has an excellent ability as a reagent for inversion of steric configuration with respect to alcohol as a raw material. That is, it makes it possible to prepare a carboxylic acid ester or alcohol having an inverted steric configuration with respect to alcohol serving as a raw material. Further, after the reaction, it is easy to separate a desired compound from a compound derived from the reagent, and the process is highly safe since there is no concern about explosion. Accordingly, it is very useful from an industrial viewpoint.

The compound represented by the general formula (6) is represented by the following general formula (7), for example.

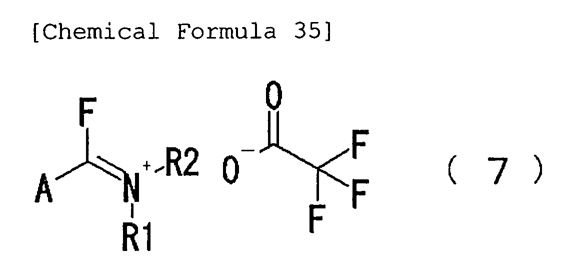

(wherein R1, R2 and A each have the same meanings as defined above.)

The compound represented by the general formula (9) is represented by the following general formulae (10) to (12), for example.

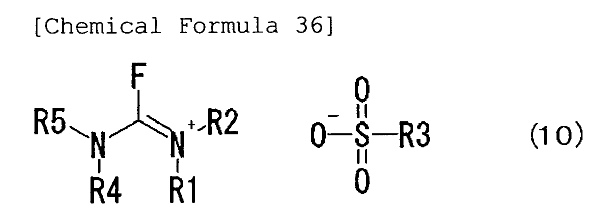

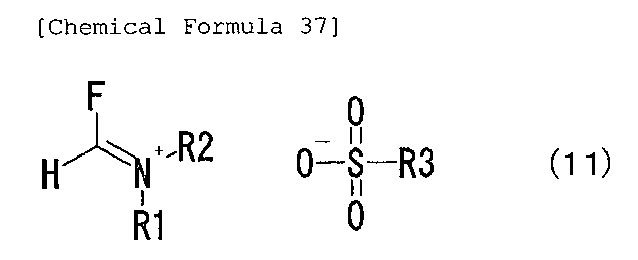

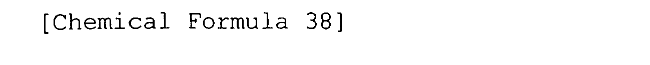

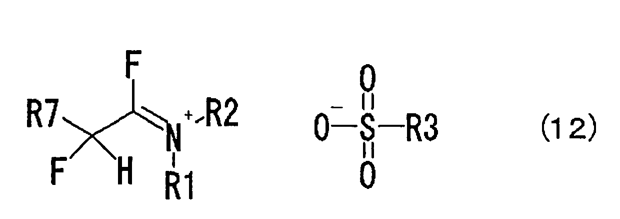

(in the general formulae (10) to (12), R1 to R5 each have the same meanings as defined above. R7 represents a fluorine atom, or an alkyl group having 1 to 6 carbon atoms, that is fully substituted with fluorine atoms.)

In the general formula (10), preferably, R1, R2, R4, and R5 are each independently an alkyl group having 1 to 6 carbon atoms. Further, the compound represented by the general formula (10) is represented by the following general formula (13), for example. 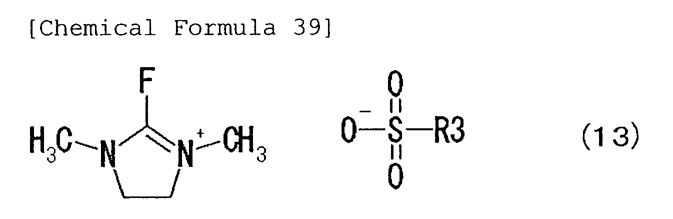 (wherein R3 has the same meaning as defined above.)

In the general formula (12), the "alkyl group having 1 to 6 carbon atoms, that is fully substituted with fluorine atoms" of R7 refers to an alkyl group having 1 to 6 carbon atoms, in which all of the hydrogen atoms are substituted with fluorine atoms. Examples of the alkyl group having 1 to 6 carbon atoms include a methyl group, an ethyl group, an isopropyl group, an n-butyl group, a tert-butyl group, an n-pentyl group, an n-hexyl group, a cyclohexyl group, a vinyl group, an allyl group, and the like.

Herein, the compound represented by the general formula (9) can be simply prepared by reacting a compound represented by the following general formula (14) with a compound represented by the following general formula (15). 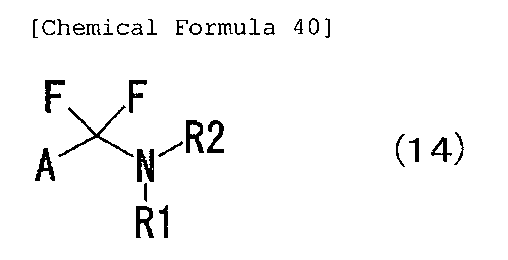 (wherein R1, R2, and A each have the same meanings as defined above. Further, any one of the fluorine atoms on the carbon atoms bonded with the nitrogen atoms may be anions to form ion-pairs.)

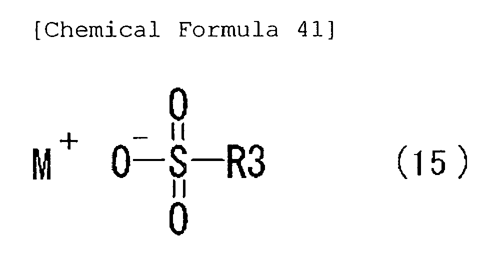

(wherein R3 has the same meaning as defined above. M⁺ represents an alkali metal ion or an alkaline earth metal ion.)

In the general formula (14), "any one of the fluorine atoms may be anions to form ion-pairs" means that the compound represented by the general formula (14) is a compound represented by the following general formula (20), in which any one of the fluorine atoms is an anion to form an ion-pair. 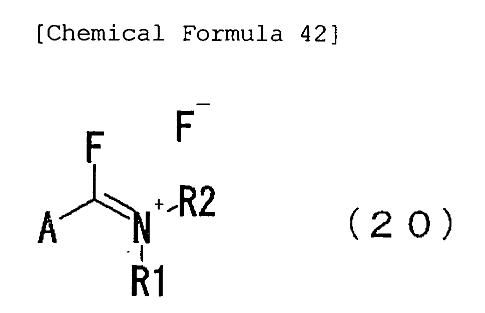 (wherein R1, R2, and A each have the same meanings as defined in the general formula (1))

Preferably, in the general formula (14), R1, R2, R3, and R5 are each independently an alkyl group having 1 to 6 carbon atoms. The "alkyl group having 1 to 6 carbon atoms" means, for example, a methyl group, an ethyl group, an isopropyl group, an n-butyl group, a tert-butyl group, an n-pentyl group, an n-hexyl group, a cyclohexyl group, a vinyl group, an allyl group, or the like.

Examples of the alkali metal ion in the general formula (15) include a lithium ion, a sodium ion, a potassium ion, and a cesium ion. Examples of the alkaline earth metal ion in the general formula (15) include a magnesium ion, a calcium ion, and a barium ion.

Furthermore, the compound represented by the general formula (14) is represented by the following general formulae (16) to (19), for example.

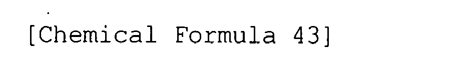

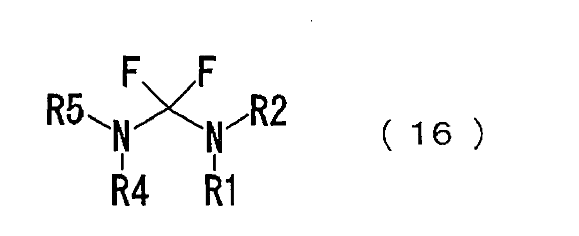

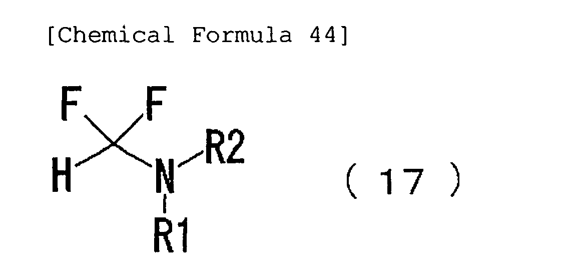

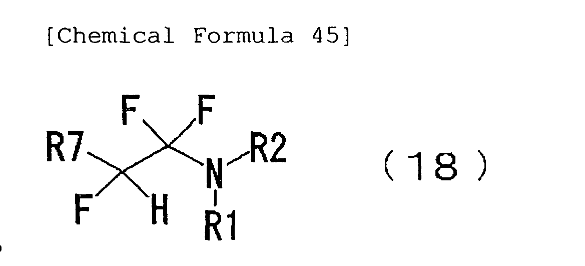

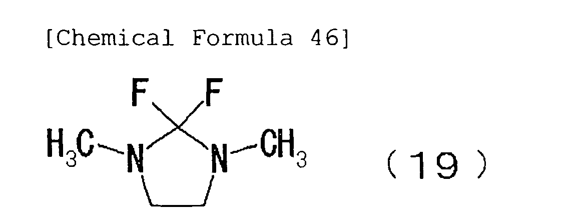

In the general formulae (16) to (19), R1, R2, R4, R5, and R7 each have the same meanings as defined above. Further, any one of the fluorine atoms on the carbon atoms bonded with the nitrogen atoms may be anions to form ion-pairs. Further, in the general formula (18), the hydrogen fluoride may leave to make an olefinic compound.

In the general formula (16), "any one of the fluorine atoms may be anions to form ion-pairs" means that the compound represented by the general formula (16) is a compound represented by the following general formula (21), in which any one of the fluorine atoms is an anion to form an ion-pair.

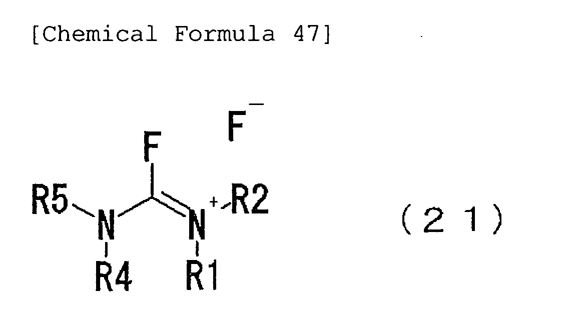

(wherein R1, R2, R4, and R5 each have the same meanings as defined above.)

In the general formula (17), "any one of the fluorine atoms may be anions to form ion-pairs" means that the compound represented by the general formula (17) is a compound represented by the following general formula (22), in which any one of the fluorine atoms is an anion to form ion-pairs.

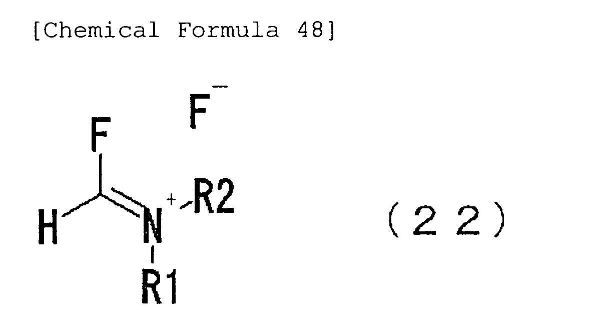

(wherein R1 and R2 each have the same meanings as defined above.)

In the general formula (18), "any one of the fluorine atoms on the carbon atoms bonded with the nitrogen atoms may be anions to form ion-pairs" means that the compound represented by the general formula (18) is a compound represented by the following general formula (23), in which any one of the fluorine atoms is an anion to form ion-pairs. 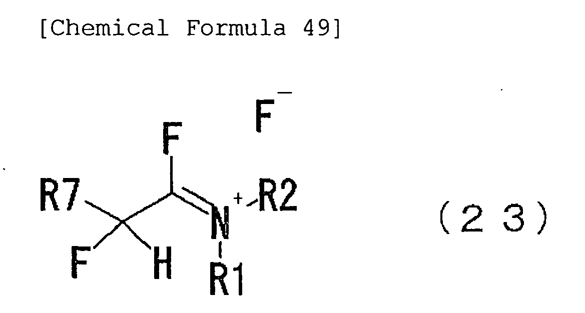 (wherein R1, R2, and R7 each have the same meanings as defined above).

In the general formula (18), "the hydrogen fluoride may leave to make an olefinic compound" means that the hydrogen fluoride leaves the compound represented by the general formula (18) to make an olefinic compound represented by the general formula (24).

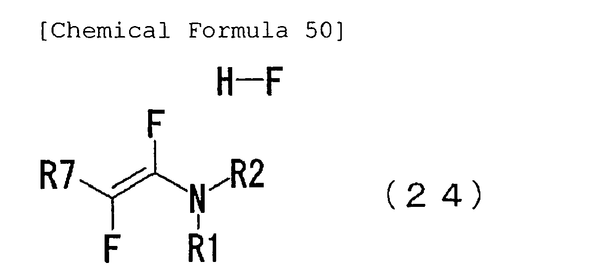

(wherein R1, R2, and R7 each have the same meanings as defined above).

The "alcohol" as used in the invention means a compound having a hydroxyl group (OH group) bonded to a chained or alicyclic hydrocarbon compound. Examples of the chained alcohol include methanol, ethanol, 1-propanol, 2-propanol, tert-butanol, octanol, benzyl alcohol, 4-phenyl-1-butanol, ethylene glycol, 2-(4'-chlorophenoxy)ethanol, (S)-2-butanol, (R)-2-hexanol, and (R)-2-octanol. Examples of the alicyclic alcohol include cyclopentanol, cyclohexanol, trans-4-tert-butylcyclohexanol, cis-4-tert-butylcyclohexanol, (R)-N-tert-butoxycarbonyl-3-hydroxypyrrolidine, (S)-3-hydroxytetrahydrofuran, and (2S,4R)-N-tert-butoxycarbonyl-4-hydroxyproline methylester.

Examples of the "optically active alcohol" as used in the invention include (S)-2-butanol, (R)-2-hexanol, (R)-2-octanol, (R)-N-tert-butoxycarbonyl-3-hydroxypyrrolidine, (S)-3-hydroxytetrahydrofuran, and (2S,4R)-N-tert-butoxycarbonyl-4-hydroxyproline methylester.

Examples of the "geometrically isomeric alcohol" as used in the invention include trans-4-tert-butylcyclohexanol, cis-4-tert-butylcyclohexanol, and trans-3-methylcyclobutanol.

Examples of the "optically active secondary alcohol" as used in the invention include (S)-2-butanol, (R)-2-hexanol, (R)-2-octanol, (R)-N-tert-butoxycarbonyl-3-hydroxypyrrolidine, (S)-3-hydroxytetrahydrofuran, and (2S,4R)-N-tert-butoxycarbonyl-4-hydroxyproline methylester.

The optically active secondary alcohol is represented by the following general formula (8), for example.

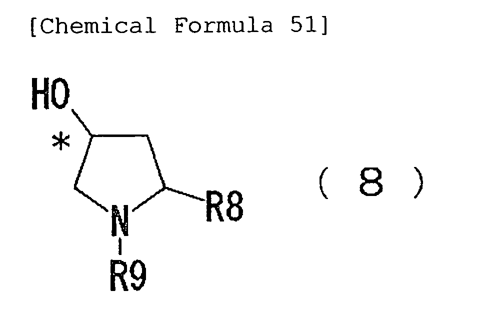

(wherein R8 represents a hydrogen atom, a carboxylic acid, a carboxylic acid ester, or a carboxylic acid amide. R9 represents a substituted or unsubstituted alkyl group, a substituted or unsubstituted aryl group, a substituted or unsubstituted alkoxycarbonyl group, a substituted or unsubstituted aryloxycarbonyl group, a substituted or unsubstituted alkylcarbonyl group, or a substituted or unsubstituted arylcarbonyl group. The symbol * represents that the hydroxyl group is optically active, indicating a compound in either an R- or an S-form.).

In the general formula (8), the "substituted or unsubstituted alkyl group" and the "substituted or unsubstituted aryl group" of R9 each have the same meanings as the "substituted or unsubstituted alkyl group" and the "substituted or unsubstituted aryl group" in the general formula (1).

In the general formula (8), the "substituted or unsubstituted alkoxycarbonyl group" of R9 means an alkoxycarbonyl group in which any hydrogen atom is substituted with a substituent, or an unsubstituted alkoxycarbonyl group. Examples of the alkoxycarbonyl group include a methoxycarbonyl group, an ethoxycarbonyl group, an isopropoxycarbonyl group, an n-butoxycarbonyl group, a tert-butoxycarbonyl group, an n-pentyloxycarbonyl group, an n-hexyloxycarbonyl group, a cyclohexyloxycarbonyl group, and an aryloxycarbonyl group. Further, examples of the substituent of the alkoxy group include a phenyl group, an alkoxy group such as a methoxy group, an ethoxy group, and a benzyloxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

In the general formula (8), the "substituted or unsubstituted aryloxycarbonyl group" of R9 means an aryloxycarbonyl group in which any hydrogen atom is substituted with a substituent, or an unsubstituted aryloxycarbonyl group. Examples of the aryloxycarbonyl group include a phenoxy group, a naphthyloxy group, and a pyridyloxy group. Further, examples of the substituent of the aryloxycarbonyl group include a phenyl group, an alkoxy group such as a methoxy group, an ethoxy group, and a benzyloxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

In the general formula (8), the "substituted or unsubstituted alkylcarbonyl group" of R9 means an alkylcarbonyl group in which any hydrogen atom is substituted with a substituent, or an unsubstituted alkylcarbonyl group. Examples of the alkylcarbonyl group include a formyl group, an acetyl group, and a pivaloyl group. Further, examples of the substituent of the alkylcarbonyl group include a phenyl group, an alkoxy group such as a methoxy group, an ethoxy group, and a benzyloxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

In the general formula (8), the "substituted or unsubstituted arylcarbonyl group" of R9 means an arylcarbonyl group in which any hydrogen atom is substituted with a substituent, or an unsubstituted arylcarbonyl group. Examples of the arylcarbonyl group include a benzoyl group, a naphthoyl group, an anthracenylcarbonyl group, a pyridylcarbonyl group, a furylcarbonyl group, and a thienylcarbonyl group. Further, examples of the substituent of the arylcarbonyl group include an alkyl group, an alkoxy group such as a methoxy group, a benzyloxy group, and a methoxyethoxy group, a phenoxy group, a nitro group, an amino group, an amide group, an alkoxycarbonyl group, a phenoxycarbonyl group, and a halogen atom such as a fluorine atom, a chlorine atom, a bromine atom, and an iodine atom.

Examples of the process for producing an ester or alcohol having an inverted steric configuration with respect to alcohol serving as a raw material, characterized by involving inversion of steric configuration, according to the present invention, are described as follows.

For example, as a process for producing an ester or alcohol having an inverted steric configuration, in a case of obtaining a sulfonic acid ester as an ester, an optically active alcohol is used as a raw material to prepare a sulfonic acid ester having an inverted steric configuration with respect to a hydroxyl group (OH group) of the raw material, as shown in (Reaction Scheme 1).

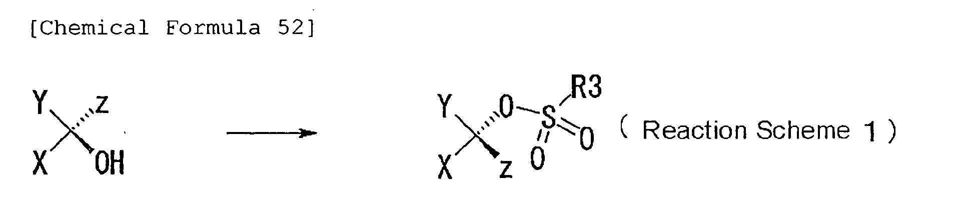

(wherein X, Y, and Z each represent different substituents. R3 represents a substituted or unsubstituted alkyl group, a substituted or unsubstituted aryl group, or a substituted or unsubstituted hetero ring.).

Further, as a process for producing an ester or alcohol having an inverted steric configuration, for example, a geometrically isomeric alcohol is used as a raw material to prepare a sulfonic acid ester having an inverted steric configuration with respect to that of a hydroxyl group (OH group) of the raw material, as described in (Reaction Scheme 2).

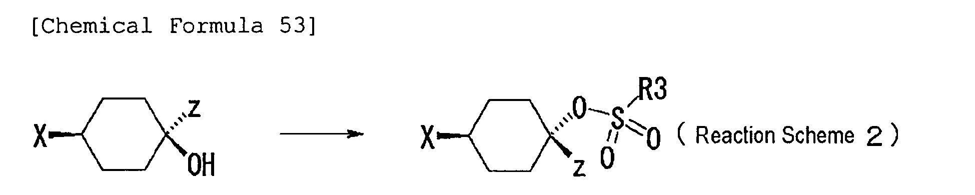

(wherein X and Z each represent different substituents. R3 has the same meaning as above.)

On the other hand, as a process for producing an ester or alcohol having an inverted steric structure, for example, in a case of obtaining a sulfonic acid ester as an ester, an optically active alcohol is used as a raw material to prepare a sulfonic acid ester having an inverted steric structure with respect to a steric configuration of a hydroxyl group (OH group) of the raw material, as described in (Reaction Scheme 3).

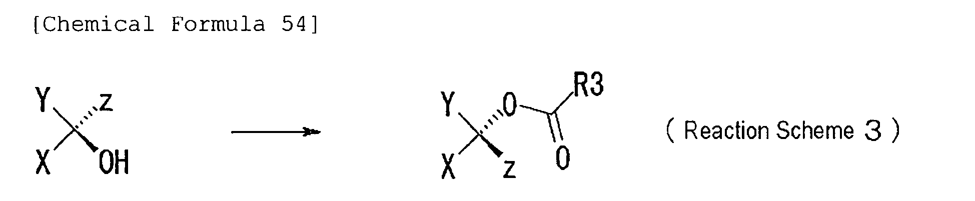

(wherein X, Y, and Z each represent different substituents. R3 has the same meaning as above.)

Alternatively, a geometrically isomeric alcohol is used as a raw material to prepare a sulfonic acid ester having an inverted steric configuration with respect to a steric configuration of a hydroxyl group (OH group) of the raw material, as described in (Reaction Scheme 4), for example.

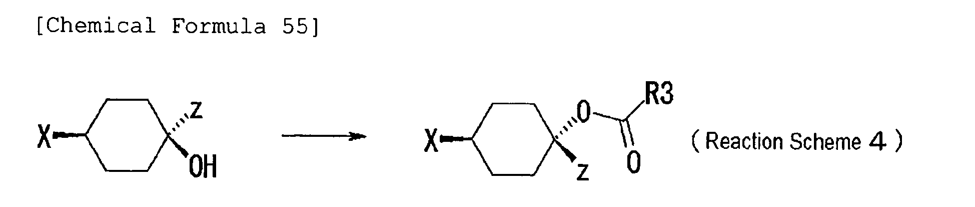

(wherein X and Z each represent different substituents. R3 has the same meaning as above.)

Further, for example, in a case of obtaining alcohol, an optically active alcohol is used as a raw material to give a carboxylic acid ester having an inverted steric configuration with respect to a steric configuration of a hydroxyl group (OH group) of the raw material as described in (Reaction Scheme 5). The process of (Reaction Scheme 5) is a preparation process using a carboxylic acid ester having an inverted steric configuration as an intermediate.

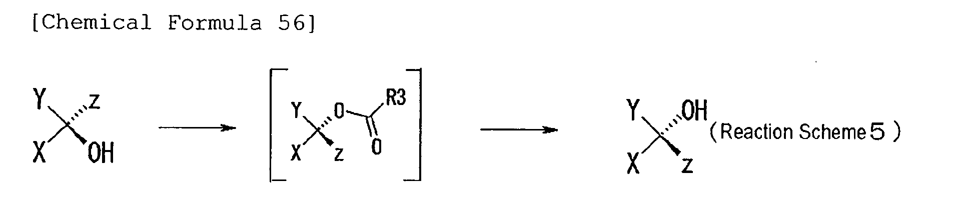

(wherein X, Y, and Z each represent different substituents. R3 has the same meaning as above.)

Alternatively, a geometrically isomeric alcohol is used as a raw material to give a carboxylic acid ester having an inverted steric configuration with respect to a steric configuration of a hydroxyl group (OH group) of the raw material as described in (Reaction Scheme 6). The process of (Reaction Scheme 6) is a preparation process using a carboxylic acid ester having an inverted steric configuration as an intermediate. 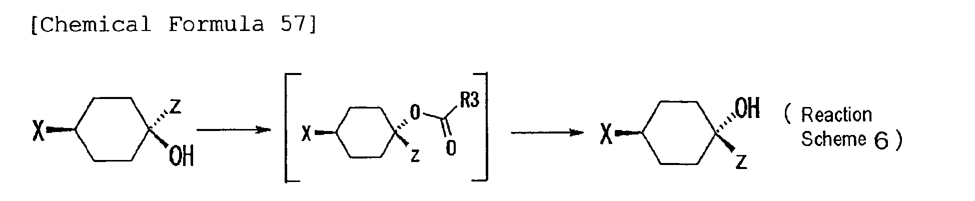 (wherein X and Z each represent different substituents. R3 has the same meaning as above.)

The process for producing the compound represented by the general formula (9) that is useful as a sulfonic acid esterifying agent is now described. The compound represented by the general formula (9) can be prepared by reacting the compound represented by the general formula (14) with the compound represented by the general formula (15). The equivalents of the compound represented by the general formula (15) to be used is not particularly limited, but it is preferably in a range of 0.5 to 3 equivalents, and more preferably, in a range of 0.9 to 1. 5 equivalents, relative to those of the compound represented by the general formula (14).

The reaction may be carried out without a solvent, but may also be carried out in a dilution solvent as needed. The reaction solvent that can be used for the reaction is not particularly limited as long as it does not adversely affect the reaction. Examples of the solvent include a hydrocarbon-based solvent such as toluene, xylene, cumene, and hexane, a halogen-based solvent such as dichloromethane and chloroform, an ether-based solvent such as diethyl ether, tetrahydrofuran, and 1,4-dioxane, an ester-based solvent such as ethyl acetate, and butyl acetate, and an aprotic polar solvent such as acetonitrile, N,N-dimethylformamide, dimethylsulfoxide, N-methylpyrrolidinone, and 1,3-dimethyl-2-imidazolidinone. Further, the solvents mentioned here may be used as a mixed solvent at an arbitrary ratio.

The reaction temperature is not particularly limited as long as the reaction proceeds, and the reaction can be carried out usually at a temperature in a range of -100°C to the boiling point of the solvent, and more preferably, in a range of -20°C to 60°C.

The compounds represented by the general formulae (10), (11), (12), and (13) can be prepared by the same treatment. Further, the compound represented by the general formula (12) may have a structure of a compound represented by the general formula (25).

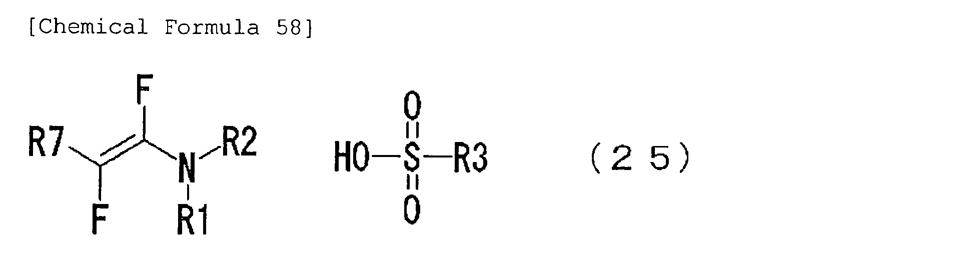

(wherein R1, R2, R3, and R7 each have the same meanings as defined above)

The process for producing a sulfonic acid ester using alcohol as a raw material is now described. A sulfonic acid ester can be prepared using alcohol as a raw material and using the compound represented by the general formula (9) as a sulfonic acid esterifying agent. In this case, the compound represented by the general formula (9) can be used in a solution form as it is without further purification, after preparation according to the preparation process. Alternatively, in other cases, it may be subject to post-treatment or purification, such as removal of a salt formed as a by-product by filtration or removal of the solvent by concentration under reduced pressure, and distillation of the compound represented by the general formula (9), after preparation according to the preparation process.

For the alcohol as a raw material, the equivalents of the sulfonic acid esterifying agent represented by the general formula (9) to be used is not particularly limited, but it is preferably in a range of 0.1 to 10 equivalents, more preferably, in a range of 0.8 to 2.0 equivalents, relative to the alcohol as a raw material.

The reaction may be carried out without a solvent, but may be carried out in a dilution solvent as needed. The reaction solvent that can be used for the reaction is not particularly limited as long as it does not adversely affect the reaction. Examples of the solvent include a hydrocarbon-based solvent such as toluene, xylene, cumene, and hexane, a halogen-based solvent such as dichloromethane and chloroform, an ether-based solvent such as diethyl ether, tetrahydrofuran, and 1,4-dioxane, an ester-based solvent such as ethyl acetate, and butyl acetate, and an aprotic polar solvent such as acetonitrile, N,N-dimethylformamide, dimethylsulfoxide, N-methylpyrrolidinone, and 1,3-dimethyl-2-imidazolidinone. Further, the solvents mentioned here may be used as a mixed solvent at an arbitrary ratio.

The reaction temperature is not particularly limited as long as the reaction proceeds, and the reaction can be carried out usually at a temperature in a range of -20°C to the boiling point of the solvent, and more preferably, in a range of 0°C to 120°C.

In this reaction, the reaction proceeds without addition of any particular base, but it can also be carried out in the coexistence of a base as needed. The base is not particularly limited, but examples thereof include organic amines such as triethylamine and pyridine.

Next, the process for preparing an ester with the involvement of inversion of steric configuration and using an optically active alcohol or a geometrically isomeric alcohol as a raw material is now described. In a case where the ester is a sulfonic acid ester, the process can be carried out by performing the same reaction as in the process for producing a sulfonic acid ester using alcohol as a raw material as above. In a case where the ester is a carboxylic acid ester, it can be prepared by using the compound represented by the general formula (6) as a carboxylic acid esterifying agent. In this case, the compound represented by the general formula (6) can be prepared by a well-known process, for example, by a process as described in Japanese Unexamined Patent Application Publication No. 2000-128868. For example, after preparing the compound represented by the general formula (6) using the compound represented by the general formula (19) and sodium trifluoroacetate in a dilution solvent, the resulting compound can be used as it is in a uniform solution state or a suspension state without carrying out particular purification. Also, in some cases, the compound can be subject to post-treatment or purification, such as removal of a salt formed as a by-product by filtration from a solution including the compound represented by the general formula (6) obtained by using the process as described above or removal of the solvent by concentration under reduced pressure, and distillation.

For the alcohol as a raw material, the equivalents of the carboxylic acid esterifying agent represented by the general formula (6) to be used is not particularly limited, but it is preferably in a range of 0.1 to 10 equivalents, more preferably, in a range of 0.8 to 3.0 equivalents, relative to the alcohol as a raw material.

The reaction may be carried out without a solvent, but may be carried out in a dilution solvent as needed. The reaction solvent that can be used for the reaction is not particularly limited as long as it does not adversely affect the reaction. Examples of the solvent include a hydrocarbon-based solvent such as toluene, xylene, cumene, and hexane, a halogen-based solvent such as dichloromethane and chloroform, an ether-based solvent such as diethyl ether, tetrahydrofuran, and 1,4-dioxane, an ester-based solvent such as ethyl acetate, and butyl acetate, and an aprotic polar solvent such as acetonitrile, N,N-dimethylformamide, dimethylsulfoxide, N-methylpyrrolidinone, and 1,3-dimethyl-2-imidazolidinone. Further, the solvents mentioned here may be used as a mixed solvent at an arbitrary ratio.

The reaction temperature is not particularly limited as long as the reaction proceeds, and the reaction can be carried out usually at a temperature in a range of -78°C to the boiling point of the solvent, more preferably, in a range of -20°C to 120°C. However, if the selectivity of inversion of steric configuration is low, in some cases, the selectivity may be improved by mixing the compound represented by the general formula (6) with alcohol as a raw material at a low temperature of about -20 to 10°C, and then warming the mixture to a temperature of 25°C or higher.

In this reaction, the reaction proceeds without addition of any particular base, but it can also be carried out in the coexistence of a base as needed. The base is not particularly limited, but examples thereof include organic amines such as triethylamine and pyridine.

Next, the process for preparing alcohol involving inversion of steric configuration with respect to an optically active alcohol or a geometrically isomeric alcohol as a raw material is now described. First, the reaction is carried out in the same manner as in the process for producing an ester using an optically active alcohol or a geometrically isomeric alcohol as a raw material and involving inversion of steric configuration as descried above. Then, the reaction solution is mixed with a basic aqueous solution to proceed a post-treatment of the carboxylic acid esterification reaction and a hydrolysis reaction of the ester group at the same time.

The aqueous basic solution is not particularly limited, but examples thereof preferably include a strongly basic aqueous solution such as an aqueous sodium hydroxide solution, an aqueous potassium hydroxide solution, an aqueous lithium hydroxide solution, an aqueous sodium carbonate solution, and an aqueous potassium carbonate solution. Further, the equivalents of the basic aqueous solution to be used is particularly not limited, but it is preferably in a range of 1 to 10 equivalents, more preferably, in a range of 2.0 to 4.0 equivalents, relative to the alcohol as a raw material.

The reaction temperature is not particularly limited as long as the reaction proceeds, but it is a temperature in a range of -78°C to the boiling point of the solvent, more preferably, in a range of -20°C to 120°C.

The inversion of steric configuration can be confirmed usually by subjecting the corresponding isomers to HPLC analysis or GC analysis, or to chiral HPLC analysis or chiral GC analysis.

A process for isolating the compound obtained in the invention is not particularly limited, but examples thereof include a process in which a reaction solution is subject to a neutralization treatment with alkaline water, and then to direct crystallization for isolation, a process in which the above-described aqueous solution that has been subject to neutralization treatment is extracted with an organic solvent, the organic solvent is then concentrated, and a crystalline compound is isolated, a process in which isolation is performed by a distillation operation from a residue obtained by concentration of the organic solvent, or a process in which isolation is performed by silica gel column chromatography from a residue obtained by concentration of the organic solvent.

Furthermore, as described herein, the raw material to be used as described in the present specification can be commercially available, or obtained by a well-known process.
The present invention is described with reference to Examples, but is not limited thereto.

### EXAMPLES

### [Example 1]

### Preparation of 2-fluoro-1,3-dimethylimidazolinium methanesulfonate (hereinafter abbreviated as FMSI)

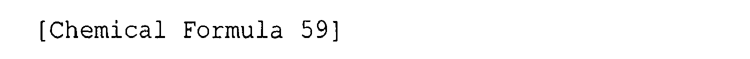

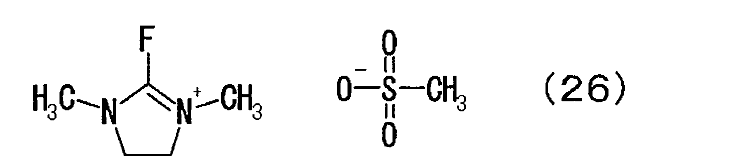

Sodium methanesulfonate (2.37 g) was suspended in acetonitrile (15 g) under a nitrogen atmosphere, and 2,2-difluoro-1,3-dimethylimidazolidine (hereinafter abbreviated as DFI) (2.73 g) was added dropwise thereto at a temperature in a range of 20 to 30°C.
The reaction solution was stirred at 20 to 30°C for 3 hours to obtain a solution of a target compound (FMSI) in acetonitrile. Yield = 100%
For analysis of the above-described reaction, a reaction was carried out in a deuterated acetonitrile solvent, and analysis was carried out by means of NMR. Sodium methanesulfonate (66 mg) was suspended in deuterated acetonitrile (2 g) under a nitrogen atmosphere, and DFI (76 mg) was added dropwise thereto at a temperature in a range of 20 to 30°C. The reaction solution was stirred at 20 to 30°C for 3 hours to obtain a solution of a target compound (FMSI) in deuterated acetonitrile. From analysis by means of NMR, a peak derived from the DFI as a raw material disappeared completely, and a new peak derived from the target compound was observed.
¹H-NMR (CD₃CN, 400 MHz) δ 3.85 (d, 4H, J=2.4 Hz), 2.96 (s, 6H), 2.42 (s, 3H)
¹³C-NMR (CD₃CN, 100 MHz) δ 159 (d, J=278 Hz), 47, 7, 39.6, 32.2

### [Example 2]

### Preparation of 2-fluoro-1,3-dimethylimidazolinium hexanesulfonate (hereinafter abbreviated as FHSI)

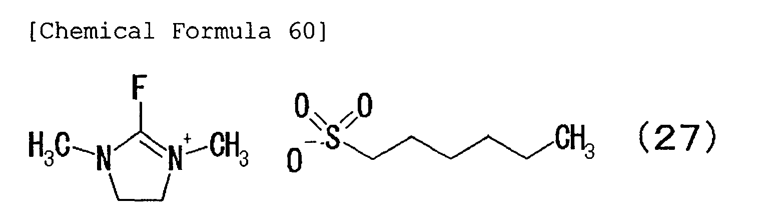

Sodium hexanesulfonate (2.96 g) was suspended in acetonitrile (18 g) under a nitrogen atmosphere, and DFI (2.14 g) was added dropwise thereto at a temperature in a range of 20 to 30°C. The reaction solution was stirred at 20 to 30°C for 3 hours to obtain a solution of a target compound (FHSI) in acetonitrile.
Yield = Quantitative
For analysis of the above-described reaction, a reaction was carried out in a deuterated acetonitrile solvent, and analysis was carried out by means of NMR. Sodium hexanesulfonate (105 mg) was suspended in deuterated acetonitrile (2 g) under a nitrogen atmosphere, and DFI (76 mg) was added dropwise thereto at a temperature in a range of 20 to 30°C. The reaction solution was stirred at 20 to 30°C for 3 hours to obtain a solution of a target compound (FHSI) in deuterated acetonitrile. From analysis by means of NMR, a peak derived from the DFI as a raw material disappeared completely, and a new peak derived from the target compound was observed.
¹H-NMR (CD₃CN, 400 MHz) δ 3.85 (d, 4H, J=2.4 Hz), 2.96 (s, 6H), 2.57-2.52 (m, 2H), 1.67-1.57 (m, 2H), 1.35-1.25 (m, 6H), 0.84 (t, 3H, J=7.6 Hz)
¹³C-NMR (CD₃CN, 100 MHz) δ 159 (d, J=279 Hz), 47, 7, 32.2, 29.2, 26.0, 23.1, 14.2

### [Example 3]

### Preparation of 2-fluoro-1,3-dimethylimidazolinium p-toluenesulfonate (hereinafter abbreviated as FTSI)

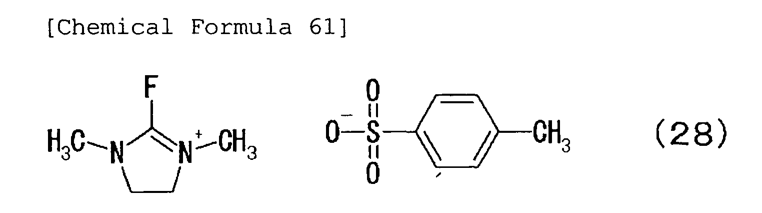

Sodium p-toluenesulfonate (3.75 g) was suspended in acetonitrile (15 g) under a nitrogen atmosphere, and 2,2-difluoro-1,3-dimethylimidazolidine (hereinafter abbreviated as DFI) (2.63 g) was added dropwise thereto at a temperature in a range of 20 to 30°C. The reaction solution was stirred at 20 to 30°C for 3 hours to obtain a solution of a target compound (FTSI) in acetonitrile.
Yield = 100%
For analysis of the above-described reaction, a reaction was carried out in a deuterated acetonitrile solvent, and analysis was carried out by means of NMR. Sodium p-toluenesulfonate (108 mg) was suspended in deuterated acetonitrile (2 g) under a nitrogen atmosphere, and DFI (76 mg) was added dropwise thereto at a temperature in a range of 20 to 30°C. The reaction solution was stirred at 20 to 30°C for 3 hours to obtain a solution of a target compound (FTSI) in deuterated acetonitrile. From analysis by means of NMR, a peak derived from the DFI as a raw material disappeared completely, and a new peak derived from the target compound was observed.
¹H-NMR (CD₃CN, 400 MHz) δ 7.75 (d, 2H, J=8.3 Hz), 7.14 (d, 2H, J=8.3 Hz), 3.81 (d, 4H, J=2.4 Hz), 2.93 (s, 6H), 2.30 (s, 3H)
¹³C-NMR (CD₃CN, 100 MHz) δ 159 (d, J=278 Hz), 145, 140, 129, 126, 47, 7, 32.2, 21.2

### [Example 4]

### Preparation of 2-fluoro-1,3-dimethylimidazolinium trifluoromethanesulfonate (hereinafter abbreviated as FTFI)

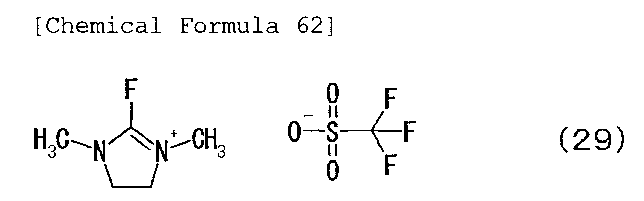

Trifluorosodium methanesulfonate (3.24 g) was suspended in acetonitrile (15 g) under a nitrogen atmosphere, and 2,2-difluoro-1,3-dimethylimidazolidine (hereinafter abbreviated as DFI) (2.56 g) was added dropwise thereto at a temperature in a range of 20 to 30°C. The reaction solution was stirred at 20 to 30°C for 3 hours to obtain a solution of a target compound (FTFI) in acetonitrile.
Yield = 100%
For analysis of the above-described reaction, a reaction was carried out in a deuterated acetonitrile solvent, and analysis was carried out by means of NMR. Trifluorosodium methanesulfonate (96 mg) was suspended in deuterated acetonitrile (2 g) under a nitrogen atmosphere, and DFI (76 mg) was added dropwise thereto at a temperature in a range of 20 to 30°C. The reaction solution was stirred at 20 to 30°C for 3 hours to obtain a solution of a target compound (FTFI) in deuterated acetonitrile. From analysis by means of NMR, a peak derived from the DFI as a raw material disappeared completely, and a new peak derived from the target compound was observed.
¹H-NMR (CD₃CN, 400 MHz) δ 3.83 (d, 4H, J=2.4 Hz), 2.96 (s, 6H)
¹³C-NMR (CD₃CN, 100 MHz) δ 159 (d, J=278 Hz), 122 (q, J=321 Hz), 47, 6, 32.1

### [Example 5]

### Preparation of tetramethyl-2-fluoroformamidinium methanesulfonate (hereinafter abbreviated as BDDFMS)

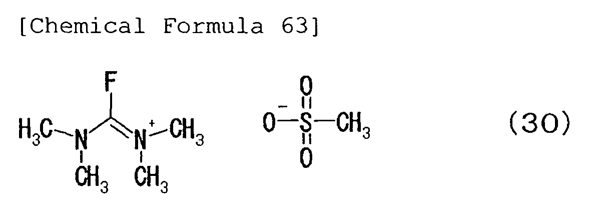

Sodium methanesulfonate (1.41 g) was suspended in acetonitrile (15 g) under a nitrogen atmosphere, and bis-dimethylamino-difluoro methane (hereinafter abbreviated as BDDF) (1. 65 g) was added dropwise thereto at a temperature in a range of 20 to 30°C. The reaction solution was stirred at 20 to 30°C for 3 hours to obtain a solution of a target compound (BDDFMS) in acetonitrile.
Yield = 100%
For analysis of the above-described reaction, a reaction was carried out in a deuterated acetonitrile solvent, and analysis was carried out by means of NMR. Sodium methanesulfonate (144 mg) was suspended in deuterated acetonitrile (2 g) under a nitrogen atmosphere, and BDDF (168 mg) was added dropwise thereto at a temperature in a range of 20 to 30°C. The reaction solution was stirred at 20 to 30°C for 3 hours to obtain a solution of a target compound (BDDFMS) in deuterated acetonitrile. From analysis by means of NMR, a peak derived from BDDF as a raw material disappeared completely, and a new peak derived from the target compound was observed.
¹H-NMR (CD₃CN, 400 MHz) δ 3.12, 3.11 (2S, 12H), 2.36 (s, 3H)
¹³C-NMR (CD₃CN, 100 MHz) δ 157 (d, J=280 Hz), 40.2, 39.6

### [Example 6]

### Preparation of N,N-diethyl-1,2,3,3,3-pentafluoropropylimidium methanesulfonate (hereinafter abbreviated as PPDAMS)

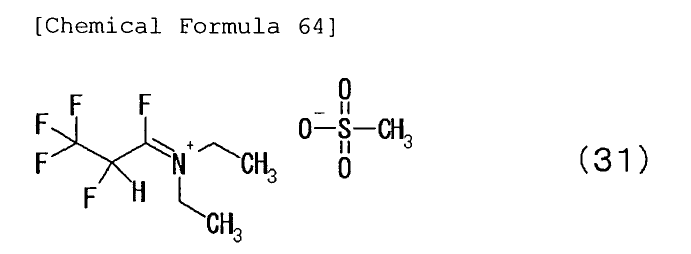

Sodium methanesulfonate (1.18 g) was suspended in acetonitrile (12 g) under a nitrogen atmosphere, and N,N-diethyl-1,1,2,3,3,3-hexafluoropropylamine (hereinafter abbreviated as PPDA) (2.22 g) was added dropwise thereto at a temperature in a range of 20 to 30°C. The reaction solution was stirred at 20 to 30°C for 3 hours to obtain a solution of a target compound (PPDAMS) in acetonitrile.
Yield = 100%
For analysis of the above-described reaction, a reaction was carried out in a deuterated acetonitrile solvent, and analysis was carried out by means of NMR. Sodium methanesulfonate (89 mg) was suspended in deuterated acetonitrile (2 g) under a nitrogen atmosphere, and PPDA (168 mg) was added dropwise thereto at a temperature in a range of 20 to 30°C. The reaction solution was stirred at 20 to 30°C for 3 hours to obtain a solution of a target compound (PPDAMS) in deuterated acetonitrile. From analysis by means of NMR, a peak derived from PPDA as a raw material disappeared completely, and a new peak derived from the target compound was observed.
¹H-NMR (CD₃CN, 400 MHz) δ 3.43-3.30 (m, 1H), 2.94 (q, 4H, J=6.8 Hz), 2.58 (s, 3H), 1.06 (t, 6H, J=6.8 Hz)

### [Example 7]

### Preparation of 4-phenylbutyl p-toluenesulfonate (hereinafter abbreviated as PBTS)

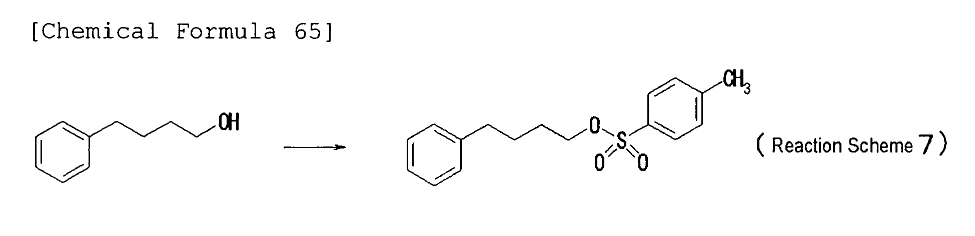

Sodium p-toluenesulfonate (3.03 g) was suspended in acetonitrile (15 g) under a nitrogen atmosphere, and DFI (2.13 g) was added dropwise thereto at a temperature in a range of 20 to 30°C. The reaction solution was stirred at 20 to 30 °C for 3 hours to obtain a solution of FTSI in acetonitrile. Subsequently, 4-phenyl-1-butanol (1. 56 g) was added to the solution, and the mixture was stirred at 60 to 65°C for 2 hours. The reaction solution was added dropwise to a solution obtained separately by suspending sodium hydrogen carbonate (3.28 g) in water (35 g). The mixture was extracted with toluene (50 g), washed with water (35 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane:ethyl acetate=20:1→10:1) to obtain a target compound (PBTS) as a colorless clear syrup.
Amount 3.10 g
Yield 98%
¹H-NMR (CD₃CN, 400 MHz) δ 7.78 (d, 2H, J=8.0 Hz), 7.33 (d, 2H, J=8.0 Hz), 7.25 (t, 2H, J=7.3 Hz), 7.17 (t, 1H, J=7.3 Hz), 7.10 (d, 2H, J=7.3 Hz), 4.03 (t, 2H, J=6.4 Hz), 2.56 (t, 2H, J=7.3 Hz), 2.44 (s, 3H), 1.70-1.60 (m, 4H)

### [Example 8]

### Preparation of 2-(4'-chlorophenoxy)ethyl p-toluenesulfonate (hereinafter abbreviated as CPETS)

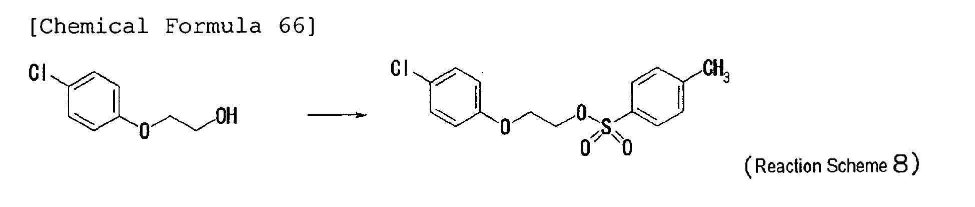

Sodium p-toluenesulfonate (3.24 g) was suspended in acetonitrile (15 g) under a nitrogen atmosphere, and DFI (2.28 g) was added dropwise thereto at a temperature in a range of 20 to 30 °C. The reaction solution was stirred at 20 to 30°C for 3 hours to obtain a solution of FTSI in acetonitrile. Subsequently, 2-(4'-chlorophenoxy)ethanol (1.92 g) was added to the solution, and the mixture was stirred at 60 to 65°C for 2 hours. The reaction solution was added dropwise to a solution obtained separately by suspending sodium hydrogen carbonate (3.51 g) in water (35 g). The mixture was extracted with toluene (50 g), washed with water (35 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 20 g, hexane:ethyl acetate=20:1→10:1), and crystallized (toluene:hexane=1:4) to obtain a target compound (CPETS) as a white crystal.
Melting point 64.0°C
Amount 2.91 g
Yield 80%
¹H-NMR (CD₃CN, 400 MHz) δ 7.80 (d, 2H, J=8.3 Hz), 7.33 (d, 2H, J=8.3Hz), 7.19 (d, 2H, J=9.3Hz), 6.70 (d, 2H, J=9.3Hz), 4.35 (dd,2H, J=4.4,5.0 Hz), 4.11 (dd,2H, J=4.4,5.0 Hz), 2.45 (s, 3H)

### [Example 9]

### Preparation of cis-4-tert-butylcyclohexyl p-toluenesulfonate (hereinafter abbreviated as cis-TBCTS)

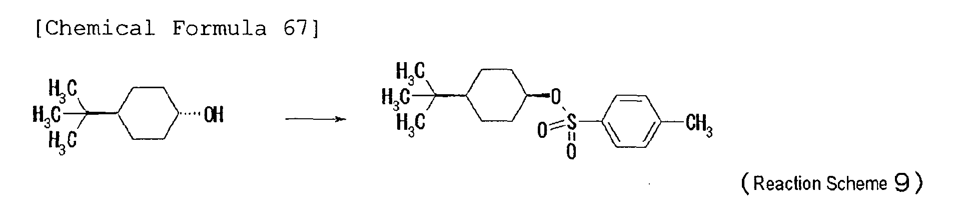

Sodium p-toluenesulfonate (1.45 g) was suspended in acetonitrile (5 g) under a nitrogen atmosphere, and DFI (1.20 g) was added dropwise thereto at a temperature in a range of 20 to 30°C. The reaction solution was stirred at 20 to 30°C for 3 hours to obtain a solution of FTSI in acetonitrile. Subsequently, trans-4-tert-butylcyclohexanol (579 mg) was added to the solution, and the mixture was stirred at 60 to 65°C for 2 hours. The reaction solution was added dropwise to a solution obtained separately by suspending sodium hydrogen carbonate (1.56 g) in water (20 g). The mixture was the extracted with toluene (25 g), washed with water (20 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 20 g, hexane:ethyl acetate=20:1→10:1) to obtain a target compound (cis-TBCTS) as a white crystal. The retention time of ¹H-NMR and HPLC was consistent with that of cis-TBCTS obtained in Reference Example 1.
Melting point 62.5°C
Amount 768 mg
Yield 83%
¹H-NMR (CD₃CN, 400 MHz) δ 7.79 (d, 2H, J=8.3 Hz), 7.32 (d, 2H, J=8. 3 Hz), 4.74 (bs, 1H), 2.44 (s, 3H), 1. 92 (bd, 2H, J=4, 2 Hz), 1. 54 (bd, 2H, J=4.2 Hz), 1.45-1.30 (m, 4H), 1.05-0.90 (m, 1H), 0.83 (s, 9H)
The stereoselectivity in the reaction was determined by analysis of the reaction solution, and using the peak area ratio of cis-TBCTS and trans-4-tert-butylcyclohexyl p-toluenesulfonate (hereinafter abbreviated as trans-TBCTS).
cis-TBCTS:trans-TBCTS=99.0:1.0
HPLC analysis condition-1
Column used YMC-PACK ODS AM-312
Eluent CH₃CN/10 mM NaH₂PO₄=75:25
Column temperature 40°C
Flow rate 1 ml/min
Detection wavelength 210 nm

### [Example 10]

### Preparation of trans-4-tert-butylcyclohexyl p-toluenesulfonate (hereinafter abbreviated as trans-TBCTS)

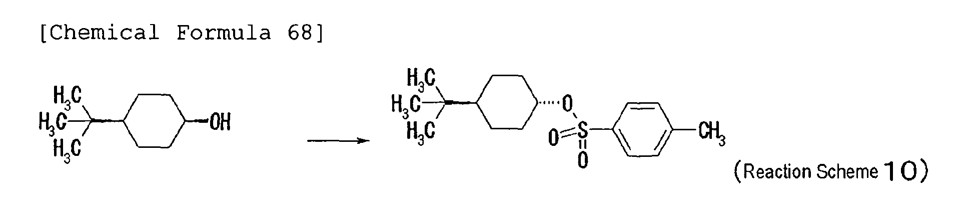

Trans-TBCTS was obtained as a white crystal from cis-4-tert-butylcyclohexanol (579 mg) by carrying out the same treatment as in Example 9. The retention time of ¹H-NMR and HPLC was consistent with that of the trans-TBCTS obtained in Reference Example 2.
Melting point 71.0°C
Amount 768 mg
Yield 83%
¹H-NMR (CD₃CN, 400 MHz) δ 7.79 (d, 2H, J=8.3 Hz), 7.32 (d, 2H, J=8.3 Hz), 4.35 (bs,1H), 2.44 (s, 3H), 2.05-1.85 (m, 2H), 1.80-1.70 (m, 2H), 1.45-1.35 (m, 2H), 1.05-0.90 (m, 3H), 0.81 (s, 9H)
The stereoselectivity in the reaction was determined by analysis of the reaction solution, and using the peak area ratio of cis-TBCTS and trans-TBCTS.
cis-TBCTS:trans-TBCTS=0.8:99.2
The HPLC condition was set as in the HPLC analysis condition-1 of Example 9.

### [Example 11]

### Preparation of (2S,4S)-N-tert-butoxycarbonyl-4-methanesulfonyloxypyrrolidine-2-carboxylic acid methylester (hereinafter abbreviated as cis-MSBPM)

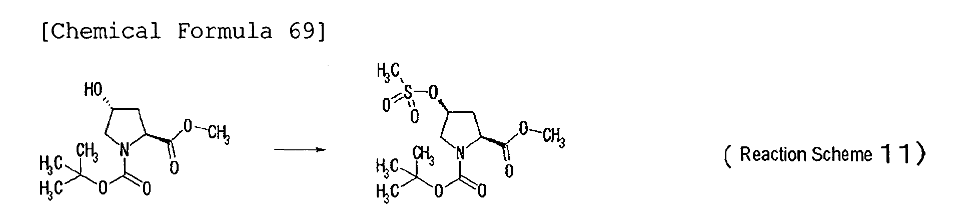

Sodiummethanesulfonate (2. 37 g) was suspended in acetonitrile (15 g) under a nitrogen atmosphere, and DFI (2.73 g) was added dropwise thereto at a temperature in a range of 20 to 30°C. The reaction solution was stirred at 20 to 30°C for 3 hours to obtain a solution of FMSI in acetonitrile. Subsequently, trans-N-tert-butoxycarbonyl-4-hydroxy-L-proline methylester (3.27 g) was added to the solution, and the mixture was stirred at 60 to 65°C for 8 hours. Apart from this, the reaction solution was added dropwise to a solution obtained by suspending sodium hydrogen carbonate (4.21 g) in water (42 g). The mixture was then extracted with toluene (50 g), washed with water (40 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 20 g, hexane:ethyl acetate=2:1→1:1) to obtain a target compound (cis-MSBPM) as a syrup. The retention time of ¹H-NMR and HPLC was consistent with that of the cis-MSBPM obtained in Reference Example 3.
Amount 3.54 g
Yield 82%
¹H-NMR (CD₃CN, 400 MHz) δ 5.23 (bs,1H), 4.55-4.45 (m, 1H), 3.82-3.75 (m, 2H), 3.76 (s, 3H), 3.02 (s, 3H), 2.56-2.48 (m, 2H), 1.48,1.43 (2s, 9H)
The stereoselectivity in the reaction was determined by analysis of the reaction solution, and using the peak area ratio of cis-MSBPM and (2S,4R)-N-tert-butoxycarbonyl-4-methanesulfonyloxypyrrolidine-2-carboxylic acid methylester (hereinafter abbreviated as trans-MSBPM).
cis-MSBPM:trans-MSBPM=99.6:0.4
HPLC analysis condition-2
Column used YMC-PACK ODS AM-312
Eluent CH₃CN/10 mM NaH₂PO₄=40/60
Column temperature 40°C
Flow rate 1 ml/min
Detection wavelength 210 nm

### [Example 12]

### Preparation of trans-MSBPM

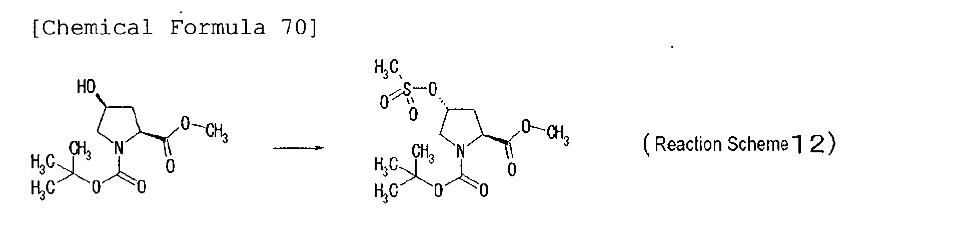

Trans-MSBPM was obtained as a white crystal from cis-N-tert-butoxycarbonyl-4-hydroxy-L-proline methylester (250 mg) by carrying out the same treatment as in Example 11. The retention time of ¹H-NMR and HPLC was consistent with that of the trans-MSBPM obtained in Reference Example 4.
Melting point 77.0°C
Amount 280 mg
Yield 85%
¹H-NMR (CD₃CN, 400 MHz) δ 7.79 (d, 2H, J=8.3 Hz), 7.32 (d, 2H, J=8.3 Hz), 4.35 (bs,1H), 2.44 (s, 3H), 2.05-1.85 (m, 2H), 1.80-1.70 (m, 2H), 1.45-1.35 (m, 2H), 1.05-0.90 (m, 3H), 0.81 (s, 9H)
The stereoselectivity in the reaction was determined by analysis of the reaction solution, and using the peak area ratio of cis-MSBPM and trans-MSBPM.
cis-MSBPM:trans-MSBPM=0.5:99.5
The HPLC condition was set as in the HPLC analysis condition-2 of Example 11.

### [Example 13]

### Preparation of cis-MSBPM

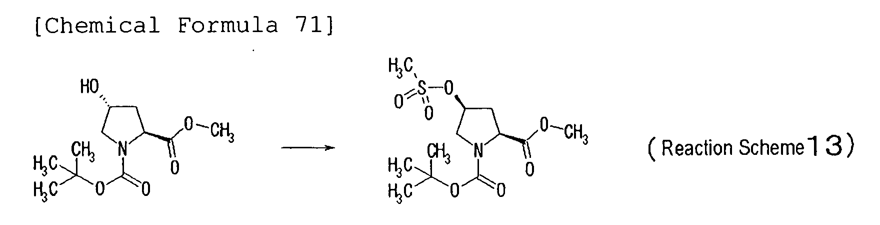

Sodium methanesulfonate (1.80 g) was suspended in acetonitrile (20 g) under a nitrogen atmosphere, and DFI (2.11 g) was added dropwise thereto at a temperature in a range of 20 to 30°C. The reaction solution was stirred at 20 to 30°C for 3 hours to obtain a solution of BDFFMS in acetonitrile. Subsequently, trans-N-tert-butoxycarbonyl-4-hydroxy-L-proline methylester (2. 50 g) was added to the solution, and the mixture was stirred at 75 to 80°C for 18 hours. The reaction solution was added dropwise to a solution obtained separately by suspending sodium hydrogen carbonate (3.21 g) in water (35 g). The mixture was then extracted with toluene (50 g), washed with water (35 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane:ethyl acetate=2:1→1:1) to obtain a target compound (cis-MSBPM) as a syrup. The retention time of ¹H-NMR and HPLC was consistent with that of the cis-MSBPM obtained in Example 11.
Amount 0.75 g
Yield 23%
The stereoselectivity in the reaction was determined by analysis of the reaction solution, and using the peak area ratio of cis-MSBPM and trans-MSBPM.
cis-MSBPM:trans-MSBPM=98:2
The HPLC condition was set as in the HPLC analysis condition-2 of Example 11.

### [Example 14]

### Preparation of cis-MSBPM

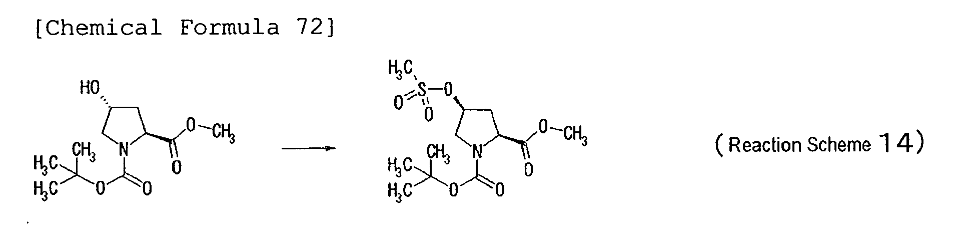

Sodium methanesulfonate (1.41 g) was suspended in acetonitrile (16 g) under a nitrogen atmosphere, and PPDA (2.66 g) was added dropwise thereto at a temperature in a range of 0 to 5°C. The reaction solution was stirred at 20 to 30°C for 3 hours to obtain a solution of PPDAMS in acetonitrile. Subsequently, trans-N-tert-butoxycarbonyl-4-hydroxy-L-proline methylester (2.50 g) was added to the solution, and the mixture was stirred at 20 to 30°C for 4 hours. The reaction solution was added dropwise to a solution obtained separately by suspending sodium hydrogen carbonate (2. 50 g) in water (25 g). The mixture was then extracted with toluene (40 g), washed with water (25 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane:ethyl acetate=2:1→1:1) to obtain a target compound (cis-MSBPM) as a syrup. The retention time of ¹H-NMR and HPLC was consistent with that of the cis-MSBPM obtained in Example 11.
Amount 1.92 g
Yield 75%
The stereoselectivity in the reaction was determined by analysis of the reaction solution, and using the peak area ratio of cis-MSBPM and trans-MSBPM.
cis-MSBPM:trans-MSBPM=99.0:1.0 The HPLC condition was set as in the HPLC analysis condition-2 of Example 11.

### [Example 15]

### Preparation of (2S,4S)-N-tert-butoxycarbonyl-4-hexanesulfonyloxypyrrolidine-2-c arboxylic acid methylester (hereinafter abbreviated as cis-HSBPM)

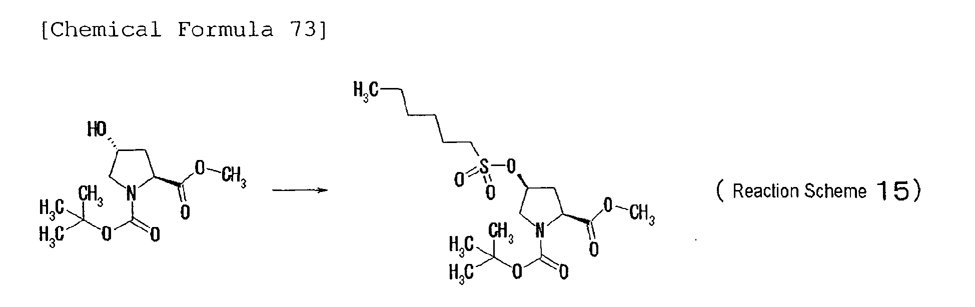

Sodium hexanesulfonate (2.96 g) was suspended in acetonitrile (18 g) under a nitrogen atmosphere, and DFI (2. 14 g) was added dropwise thereto at a temperature in a range of 20 to 30°C. The reaction solution was stirred at 20 to 30°C for 3 hours to obtain a solution of FMSI in acetonitrile. Subsequently, trans-N-tert-butoxycarbonyl-4-hydroxy-L-proline methylester (2.57 g) was added to the solution, and the mixture was stirred at 60 to 65°C for 7 hours. The reaction solution was added dropwise to a solution obtained separately by suspending sodium hydrogen carbonate (3. 30 g) in water (33 g). The mixture was then extracted with toluene (50 g), washed with water (33 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane: ethyl acetate=5:1→4:1→3:1) to obtain a target compound (cis-HSBPM) as a colorless clear syrup. The retention time of ¹H-NMR and HPLC was consistent with that of the cis-HSBPM obtained in Reference Example 5.
Amount 3.31 g
Yield 80%
¹H-NMR (CD₃CN, 400 MHz) δ 5.23 (bs, 1H), 4.51,4.40 (2bt, 1H, J=5.0 Hz), 3.78-3.74 (m, 2H), 3.75 (s, 3H), 3.07 (bt, 2H J=7.8 Hz), 2.54 (bs, 1H), 2.49 (bs, 1H), 1.85-1.78 (m, 2H), 1.50-1.40 (m, 2H), 1.48,1.43 (2s, 9H), 1.35-1.30 (m, 4H), 0.90 (t, 3H, J=6.9 Hz)
The stereoselectivity in the reaction was determined by analysis of the reaction solution, and using the peak area ratio of cis-HSBPM and
(2S,4R)-N-tert-butoxycarbonyl-4-hexanesulfonyloxypyrrolidine-2-c arboxylic acid methylester (hereinafter abbreviated as trans-HSBPM).
cis-HSBPM:trans-HSBPM=99.4:0.6
HPLC analysis condition-3
Column used YMC-PACK ODS AM-312
Eluent CH₃CN/10 mM NaH₂PO₄=60/40
Column temperature 40°C
Flow rate 1 ml/min
Detection wavelength 210 nm

### [Example 16]

### Preparation of trans-HSBPM)

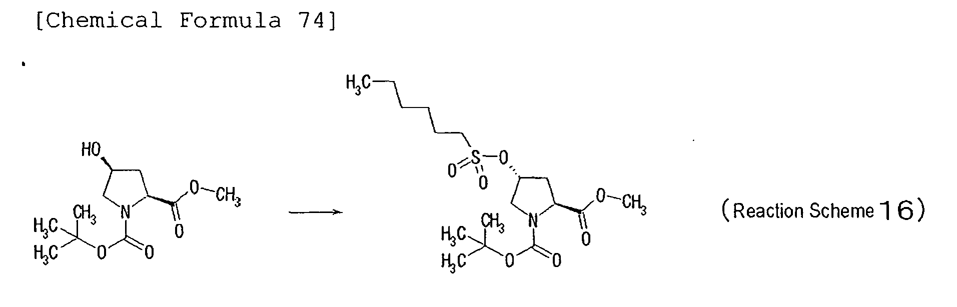

Trans-HSBPM was obtained as a colorless clear syrup from cis-N-tert-butoxycarbonyl-4-hydroxy-L-proline methylester (250 mg) by carrying out the same treatment as in Example 13. The retention time of ¹H-NMR and HPLC was consistent with that of the trans-HSBPM obtained in Reference Example 6.
Amount 315 mg
Yield 78%
¹H-NMR (CD₃CN, 400MHz) δ 5.25 (bs, 1H), 4.46,4.39 (2t, 1H, J=7.8 Hz), 3.86-3.72 (m, 2H), 3.75 (s, 3H), 3.13-3.09 (m, 2H), 2.68-2.52 (m, 1H), 2.30-2.20 (m, 1H), 1.88-1.78 (m, 2H), 1.45-1.40 (m, 2H), 1.47,1.42 (2s, 9H), 1.35-1.25 (m, 4H), 0.90 (t, 3H, J=6.8 Hz)
The stereoselectivity in the reaction was determined by analysis of the reaction solution, and using the peak area ratio of cis-HSBPM and trans-HSBPM.
cis-HSBPM:trans-HSBPM=0.5:99.5
The HPLC condition was set as in the HPLC analysis condition-3 of Example 15.

### [Example 17]

### Preparation of (2S,4S)-N-tert-butoxycarbonyl-4-p-toluenesulfonyloxypyrrolidine-2-carboxylic acid methylester (hereinafter abbreviated as cis-TSBPM)

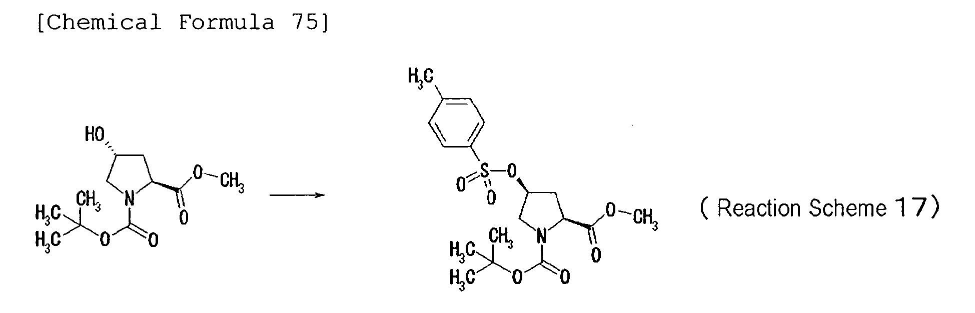

Sodium p-toluenesulfonate (3.75 g) was suspended in acetonitrile (15 g) under a nitrogen atmosphere, and DFI (2.63 g) was added dropwise thereto at a temperature in a range of 20 to 30°C. The reaction solution was stirred at 20 to 30°C for 3 hours to obtain a solution of FTSI in acetonitrile. Subsequently, trans-N-tert-butoxycarbonyl-4-hydroxy-L-proline methylester (3.15 g) was added to the solution, and the mixture was stirred at 60 to 65°C for 3 hours. The reaction solution was added dropwise to a solution obtained separately by suspending sodium hydrogen carbonate (4.05 g) in water (40 g). The mixture was then extracted with toluene (50 g), washed with water (40 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane: ethyl acetate=4:1→3:1→2:1) to obtain a target compound (cis-TSBPM) as a syrup. The retention time of ¹H-NMR and HPLC was consistent with that of the cis-TSBPM obtained in Reference Example 7.
Amount 3.85 g
Yield 75%
¹H-NMR (CD₃CN, 400 MHz) δ 7.76 (d, 2H, J=7.8 Hz), 7.35 (d, 2H, J=7.8 Hz), 5.10-5.00 (m, 1H), 4.45-4.30 (m, 1H), 3.70-3.55 (m, 2H), 3.69 (s, 3H), 2.50-2.35 (m, 2H), 2.45 (s, 3H), 1.44,1.40 (2s, 9H)
The stereoselectivity in the reaction was determined by analysis of the reaction solution, and using the peak area ratio of cis-TSBPM and
(2S,4R)-N-tert-butoxycarbonyl-4-p-toluenesulfonyloxypyrrolidine-2-carboxylic acid methylester (hereinafter abbreviated as trans-TSBPM).
cis-TSBPM:trans-TSBPM=99.2:0.8
HPLC analysis condition-4
Column used YMC-PACK ODS AM-312
Eluent CH₃CN/10 mM NaH₂PO₄=50/50
Column temperature 40°C
Flow rate 1 ml/min
Detection wavelength 210 nm

### [Example 18]

### Preparation of trans-TSBPM

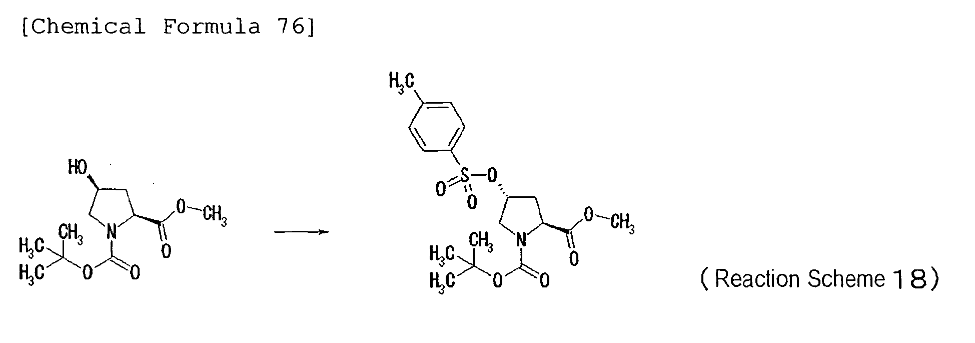

Trans-TSBPM was obtained as a white crystal from cis-N-tert-butoxycarbonyl-4-hydroxy-L-proline methylester (250 mg) by carrying out the same treatment as in Example 17. The retention time of ¹H-NMR and HPLC was consistent with that of the trans-TSBPM obtained in Reference Example 8.
Melting point 72.5°C
Amount 338 mg
Yield 83%
¹H-NMR (CD₃CN, 400 MHz) δ 7.78 (d, 2H, J=8.3 Hz), 7.36 (d, 2H, J=8.3 Hz), 5.10-5.00 (m, 1H), 4.40-4.33 (m, 1H), 3.72 (s, 3H), 3.65-3.55 (m, 2H), 2.57-2.37 (m, 1H), 2.46 (s, 3H), 2.20-2.05 (m, 1H), 1.42,1.39 (2s, 9H)
The stereoselectivity in the reaction was determined by analysis of the reaction solution, and using the peak area ratio of cis-TSBPM and trans-TSBPM.
cis-TSBPM:trans-TSBPM=0.6:99.4
The HPLC condition was set as in the HPLC analysis condition-4 of Example 17.

### [Example 19]

### Preparation of (S)-N-tert-butoxycarbonyl-3-methanesulfonyloxypyrrolidine (hereinafter abbreviated as S-BMSP)

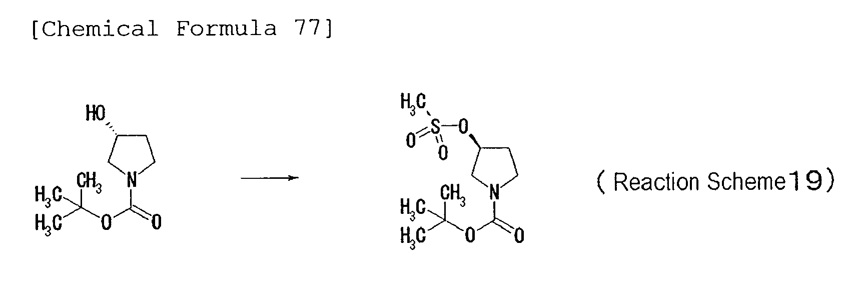

Sodium methanesulfonate (2.28 g) was suspended in acetonitrile (15 g) under a nitrogen atmosphere, and DFI (2. 62 g) was added dropwise thereto at a temperature in a range of 20 to 30°C. The reaction solution was stirred at 20 to 30°C for 3 hours to obtain a solution of FMSI in acetonitrile. Subsequently, (R)-N-tert-butoxycarbonyl-3-hydroxypyrrolidine (2.41 g) was added to the solution, and the mixture was stirred at 60 to 65 °C for 7 hours. The reaction solution was added dropwise to a solution obtained separately by suspending sodium hydrogen carbonate (4.05 g) in water (40 g). The mixture was then extracted with toluene (50 g), washed with water (40 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane:ethyl acetate=3:1→2:1) to obtain a target compound (S-BMSP) as a colorless clear syrup. The results of ¹H-NMR, and derivatization and analysis of optically active HPLC columns in accordance with Reference Example 12 were the same as in S-BMSP obtained in Reference Example 9.
Amount 2.74 g
Yield 80%
¹H-NMR (CD₃CN, 400 MHz) δ 5.28-5.24 (m, 1H), 3.68-3.46 (m, 4H), 3.05 (s, 3H), 2.28-2.12 (m, 2H), 1.47 (s, 9H)
The stereoselectivity in the reaction was determined by derivation of (R)-3-phthalimide-N-tert-butoxycarbonylpyrrolidine (hereinafter abbreviated as R-BPP) as in Reference Example 12, and using the peak area ratio of R-BPP and
(S)-3-phthalimide-N-tert-butoxycarbonylpyrrolidine (hereinafter abbreviated as S-BPP) under an HPLC analysis condition-5.
S-BMSP:R-BMSP=99.5:0.5

### [Example 20]

### Preparation of R-BMSP

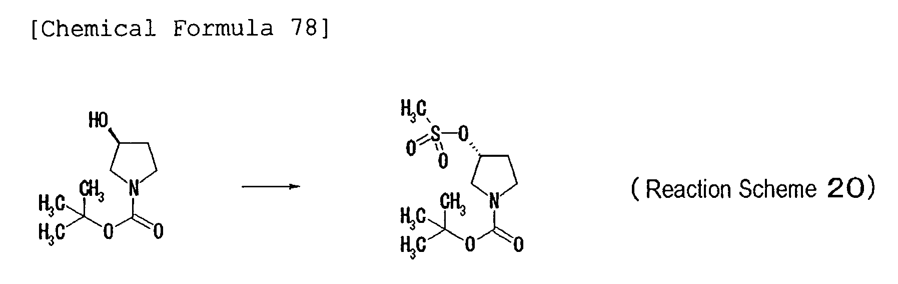

A target compound (R-BMSP) was obtained as colorless clear syrup from (S)-N-tert-butoxycarbonyl-3-hydroxypyrrolidine (2.41 g) by carrying out the same treatment as in Example 19. ¹H-NMR was consistent with that of the S-BMSP obtained in Example 19.
Amount 2.81 g
Yield 82%
The stereoselectivity in the reaction was determined by derivation as in Reference Example 12, and using the peak area ratio of R-BPP and S-BPP under the HPLC analysis condition-5.
S-BMSP:R-BMSP=0.5:99.5

### [Example 21]

### Preparation of S-BMSP

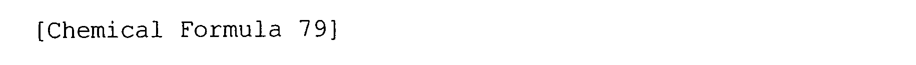

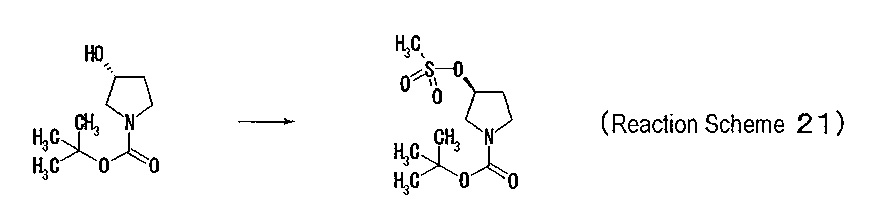

Sodium methanesulfonate (1.41 g) was suspended in acetonitrile (15 g) under a nitrogen atmosphere, and BDDF (1.65 g) was added dropwise thereto at a temperature in a range of 20 to 30°C. The reaction solution was stirred at 20 to 30°C for 3 hours to obtain a solution of BDDFMS in acetonitrile. Subsequently, (R)-N-tert-butoxycarbonyl-3-hydroxypyrrolidine (1.49 g) was added to the solution, and the mixture was stirred at 75 to 80°C for 8 hours. The reaction solution was added dropwise to a solution obtained separately by suspending sodium hydrogen carbonate (2.50 g) in water (25 g). The mixture was then extracted with toluene (30 g), washed with water (20 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 50 g, hexane:ethyl acetate=3:1→2:1) to obtain a target compound (S-BMSP) as a colorless clear syrup. ¹H-NMR was consistent with that of the S-BMSP obtained in Example 19.
Amount 1.37 g
Yield 65%
The stereoselectivity in the reaction was determined by derivation as in Reference Example 12, and using the peak area ratio of R-BPP and S-BPP under an HPLC analysis condition-5.
S-BMSP:R-BMSP=99.5:0.5

### [Example 22]

### Preparation of S-BMSP

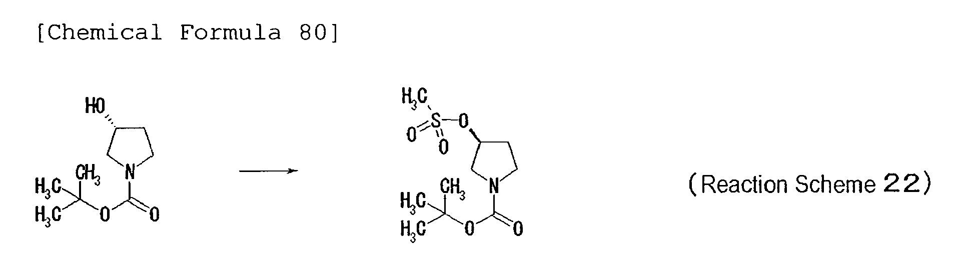

Sodium methanesulfonate (1.18 g) was suspended in acetonitrile (12 g) under a nitrogen atmosphere, and PPDA (2.22 g) was added dropwise thereto at a temperature in a range of 0 to 5°C. The reaction solution was stirred at 20 to 30°C for 3 hours to obtain a solution of PPDAMS in acetonitrile. Subsequently, (R)-N-tert-butoxycarbonyl-3-hydroxypyrrolidine (1.25 g) was added to the solution, and the mixture was stirred at 20 to 30°C for 8 hours. The reaction solution was added dropwise to a solution obtained separately by suspending sodium hydrogen carbonate (2.10 g) in water (25 g). The mixture was then extracted with toluene (30 g), washed with water (20 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 50 g, hexane:ethyl acetate=3:1→2:1) to obtain a target compound (S-BMSP) as a colorless clear syrup. ¹H-NMR was consistent with that of the S-BMSP obtained in Example 19.
Amount 1.36 g
Yield 77%
The stereoselectivity in the reaction was determined by derivation as in Reference Example 12, and using the peak area ratio of R-BPP and S-BPP under an HPLC analysis condition-5.
S-BMSP:R-BMSP=99.5:0.5

### [Example 23]

### Preparation of (S)-N-tert-butoxycarbonyl-3-hexanesulfonyloxypyrrolidine (hereinafter abbreviated as S-BHSP)

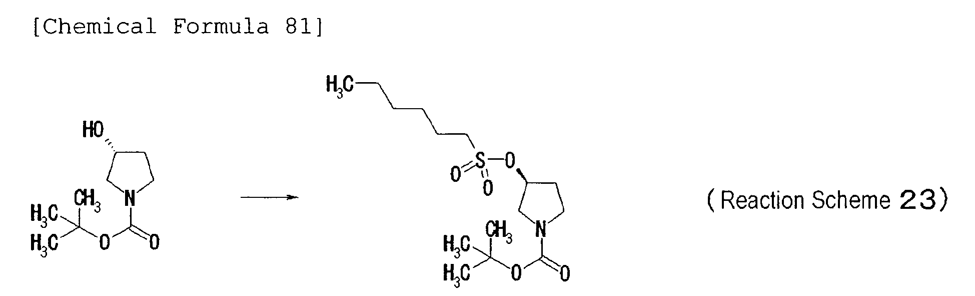

p-Sodium hexanesulfonate (2.61 g) was suspended in acetonitrile (14 g) under a nitrogen atmosphere, and DFI (1.89 g) was added dropwise thereto at a temperature in a range of 20 to 30°C. The reaction solution was stirred at 20 to 30 °C for 3 hours to obtain a solution of FHSI in acetonitrile. Subsequently, (R)-N-tert-butoxycarbonyl-3-hydroxypyrrolidine (1.74 g) was added to the solution, and the mixture was stirred at 20 to 30 °C for 8 hours. The reaction solution was added dropwise to a solution obtained separately by suspending sodium hydrogen carbonate (2.93 g) in water (30 g). The mixture was then extracted with toluene (50 g), washed with water (30 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 50 g, hexane:ethyl acetate=3:1→2:1) to obtain a target compound (S-BHSP) as a colorless clear syrup. The results of ¹H-NMR, and derivatization and analysis of optically active HPLC columns in accordance with Reference Example 12 were the same as in S-BHSP obtained in Reference Example 10.
Amount 2.74 g
Yield 83%
¹H-NMR (CD₃CN, 400 MHz) δ 5.25 (bs, 1H), 3.70-3.45 (m, 4H), 2.35-2.05 (m, 2H), 1.90-1.80 (m, 2H), 1.47 (s, 9H), 1.50-1.40 (m, 2H), 1.35-1.28 (m, 4H), 0.90 (t, 3H, J=6.8 Hz)
The stereoselectivity in the reaction was determined by derivation as in Reference Example 12, and using the peak area ratio of R-BPP and S-BPP under an HPLC analysis condition-5.
S-BHSP:(R)-N-tert-butoxycarbonyl-3-hexanesulfonyloxypyrrol idine (hereinafter abbreviated as R-BHSP)=99.5:0.5

### [Example 24]

### Preparation of R-BHSP

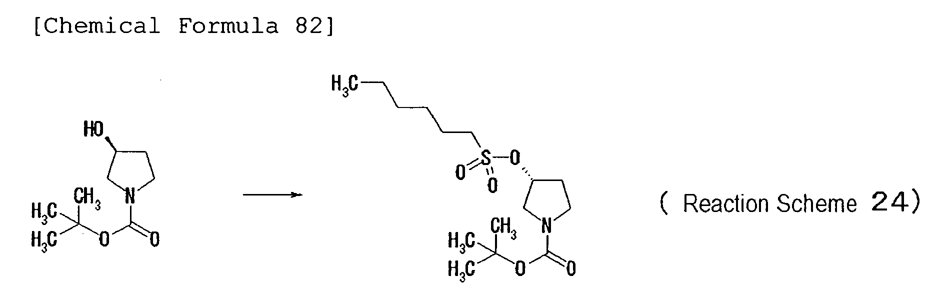

A target compound (R-BHSP) was obtained as colorless clear syrup from 3S-N-tert-butoxycarbonyl-3-hydroxypyrrolidine (1.74 g) by carrying out the same treatment as in Example 23. ¹H-NMR was consistent with that of the S-BHSP obtained in Example 23.
Amount 2.64 g
Yield 80%
The stereoselectivity in the reaction was determined by derivation as in Reference Example 12, and using the peak area ratio of R-BPP and S-BPP under the HPLC analysis condition-5.
S-BHSP:R-BHSP=0.5:99.5

### [Example 25]

### Preparation of (S)-N-tert-butoxycarbonyl-3-p-toluenesulfonyloxypyrrolidine (hereinafter abbreviated as S-BTSP)

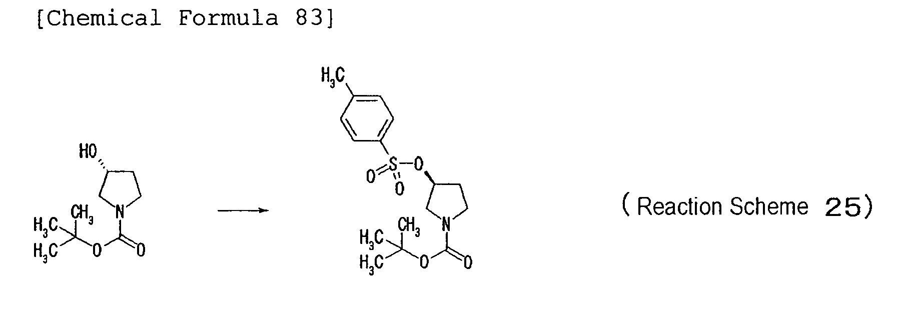

Sodium p-toluenesulfonate (3.75 g) was suspended in acetonitrile (20 g) under a nitrogen atmosphere, and DFI (2.63 g) was added dropwise thereto at a temperature in a range of 0 to 5°C. The reaction solution was stirred at 20 to 30°C for 3 hours to obtain a solution of FTSI in acetonitrile. Subsequently, (R)-N-tert-butoxycarbonyl-3-hydroxypyrrolidine (2.41 g) was added to the solution, and the mixture was stirred at 20 to 30°C for 8 hours. The reaction solution was added dropwise to a solution obtained separately by suspending sodium hydrogen carbonate (4.05 g) in water (40 g). The mixture was then extracted with toluene (50 g), washed with water (40 g), and subsequently, the obtained toluene layer was dried over anhydrous magnesium sulfate. Anhydrous magnesium sulfate was separated by filtration, and the toluene solution was concentrated under reduced pressure. The obtained residue was purified by silica gel column chromatography (silica gel 60 g, hexane:ethyl acetate=4:1→3:1) to obtain a target compound (S-BTSP) as a colorless clear syrup. The results of ¹H-NMR, and derivatization and analysis of optically active HPLC columns in accordance with Reference Example 12 were the same as in S-BTSP obtained in Reference Example 11.
Amount 3.44 g
Yield 78%
¹H-NMR (CD₃CN, 400 MHz) δ 7.79 (d, 2H, J=8.3 Hz), 7.35 (d, 2H, J=8.35), 5.04 (bs, 1H), 3.53-3.37 (m, 4H), 2.46 (s, 3H), 2.20-1.95 (m, 2H), 1.43 (s, 9H)
The stereoselectivity in the reaction was determined by derivation as in Reference Example 12, and using the peak area ratio of R-BPP and S-BPP under the HPLC analysis condition-5.
S-BTSP:(R)-N-tert-butoxycarbonyl-3-p-toluenesulfonyloxypyr rolidine (hereinafter abbreviated as R-BTSP)=99.5:0.5

### [Example 26]

### Preparation of R-BTSP

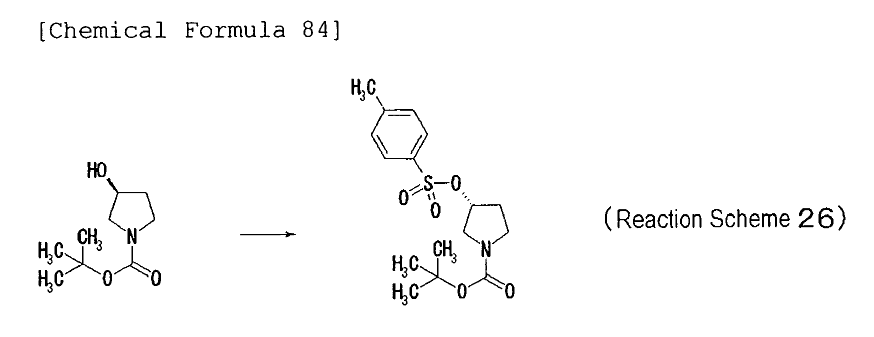

A target compound (R-BTSP) was obtained as colorless clear syrup by carrying out the same treatment as in Example 23, and from (S)-N-tert-butoxycarbonyl-3-hydroxypyrrolidine (2.41 g). ¹H-NMR was consistent with that of the S-BTSP obtained in Example 23.
Amount 3.53 g
Yield 80%
The stereoselectivity in the reaction was determined by derivation as in Reference Example 12, and using the peak area ratio of R-BPP and S-BPP under the HPLC analysis condition-5.
S-BMSP:R-BMSP=0.5:99.5

### [Example 27]

### Preparation of (S)-N-benzyloxycarbonyl-3-trifluoroacetoxypyrrolidine (hereinafter abbreviated as S-CTFAP)

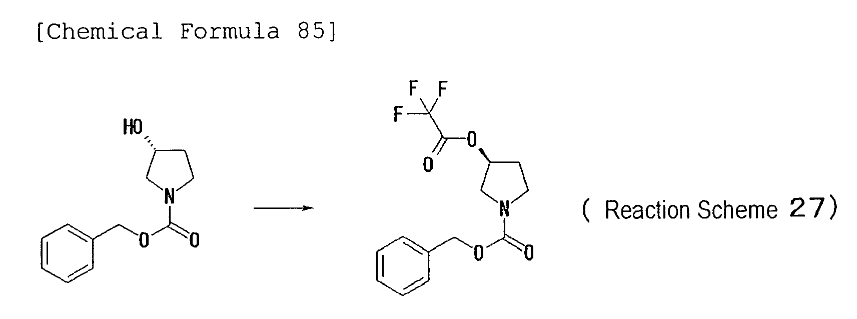

Sodium trifluoroacetate (1.34 g) was suspended in toluene (8.9 g), DFI (1.34 g) was added dropwise thereto at room temperature, and the mixture was stirred at room temperature for 1 hour. Subsequently, (R)-N-benzyloxycarbonyl-3-hydroxypyrrolidine (hereinafter abbreviated as R-CHP) (1.81 g) was added thereto. The reaction solution was stirred at room temperature for 1 hour, and then at 50 °C for 2 hours. To the reaction solution was added toluene (20 g), and the mixture was added dropwise to an ice-cooled, 5 wt% aqueous sodium hydrogen carbonate solution (25.2 g). The toluene layer was separated, and dried over anhydrous magnesium sulfate. Anhydrous magnesium sulfate was separated by filtration, and then the filtrate was concentrated under reduced pressure to obtain a target compound.
Amount 2.92 g
Yield 92%
¹H-NMR (toluene-d8, 400 MHz) δ 7.20-6.98 (m, 5H), 5.08 (d, 1H, J=11.6 Hz), 4.93 (dd,1H, J=5.2,11.6 Hz), 4.65 (bs, 1H), 3.42-3.02 (m, 4H), 1.40-1.28 (m, 1H), 1.28-1.15 (m, 1H)
The stereoselectivity in the reaction was determined by dissolving the obtained compound in toluene (10 ml), and stirring it in a 1 N-aqueous NaOH solution (20 ml) at room temperature for 30 min to derive a corresponding
(S)-N-benzyloxycarbonyl-3-hydroxypyrrolidine (hereinafter abbreviated as S-CHP), and using the peak area ratio of S-CHP and R-CHP under an HPLC analysis condition 6.
S-CTFAP:R-CTFAP=97:3
HPLC analysis condition 6
Chiral column used DAICEL CHIRALPACK AD-H
Eluent hexane/2-propanol=95/5
Flow rate 1 ml/min
Detection wavelength UV 210 nm
Column temperature 35°C

### [Example 28]

### Preparation of S-CTFAP

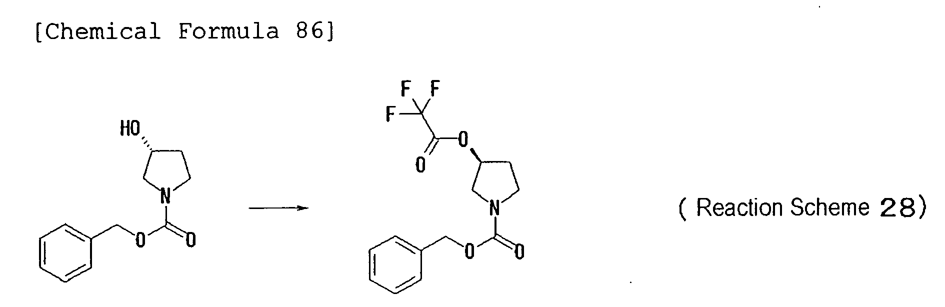

Sodium trifluoroacetate (1.34 g) was suspended in toluene (8. 9 g), and DFI (1.34 g) was added dropwise thereto at 0°C. The mixture was stirred at 0°C for 1 hour, and then R-CHP (1.81 g) was added thereto. The reaction solution was stirred at 0°C for 1 hour, and then at 50°C for 2 hours. To the reaction solution was added toluene (20 g), and the mixture was added dropwise to an ice-cooled, 5 wt% aqueous sodium hydrogen carbonate solution (25.2 g). The toluene layer was separated, and dried over anhydrous magnesium sulfate. Anhydrous magnesium sulfate was separated by filtration, and then the filtrate was concentrated under reduced pressure to obtain a target compound.
Amount 2.98 g
Yield 94%
¹H-NMR (toluene-d8, 400 MHz) was consistent with that in Example 27.
The stereoselectivity in the reaction was determined in the same method as in Example 27.
S-CTFAP:R-CTFAP=99.8:0.2

### [Example 29]

### Preparation of S-CHP

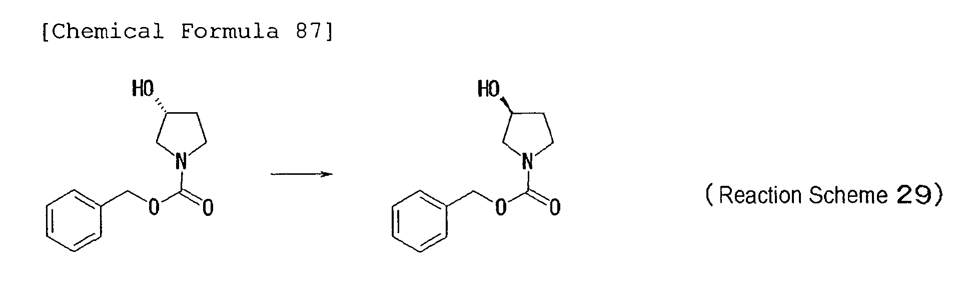

Sodium trifluoroacetate (32.6 g) was suspended in toluene (213 g), and DFI (32.6 g) was added dropwise thereto at 0°C. The mixture was stirred at 0°C for 1 hour, and then R-CHP (44.2 g) was added thereto. The reaction solution was stirred at 0°C for 1 hour, and then at 50°C for 2 hours. The reaction solution was kept at 15°C or lower, a 5 wt% aqueous sodium hydrogen carbonate solution (480 g) was added dropwise to the reaction solution, which was stirred at room temperature for 30 min. Subsequently, the toluene layer was separated. The toluene layer was washed three times with water (100 g), and then concentrated under reduced pressure. To the obtained residue were added toluene (40 g) and hexane (80 g), and the mixture was crystallized at 0°C. The obtained crystal was collected by filtration, and dried at 40°C for 12 hours under reduced pressure to obtain a target compound.
Amount 39.3 g
Yield 89%
Stereoisomer ratio S-CHP:R-CHP=99.9:0.1
¹H-NMR (toluene-d8, 400 MHz) δ 7.23-7.20 (m, 2H), 7.13-6.98 (m, 3H), 5.11-4.99 (m, 2H), 3.93 (bs, 1H), 3.46-3.12 (m, 4H), 1.65-1.45 (m, 1H), 1,45-1.30 (m, 1H)
The stereoselectivity in the reaction was determined by using the concentrated residue before crystallization and using the peak area ratio of S-CHP and R-CHP under an HPLC analysis condition 6.
S-CHP:R-CHP=99.8:0.2

### [Example 30]

### Preparation of S-CTFAP

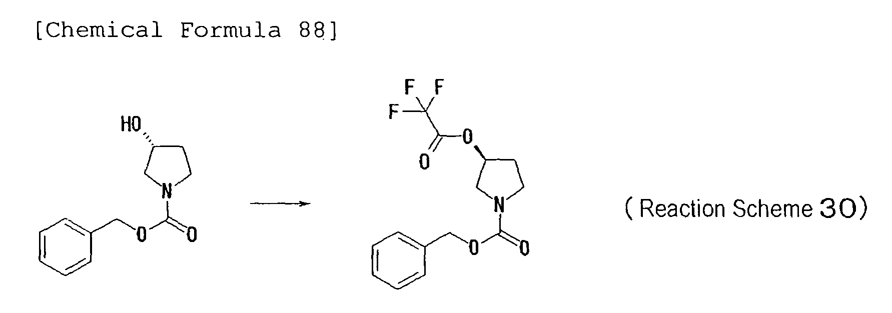

Sodium trifluoroacetate (2.04 g) was suspended in toluene (10 g), and PPDA (3.35 g) was added dropwise thereto at 0°C. The mixture was stirred at 0°C for 1 hour, and then R-CHP (1.11 g) was added thereto. The reaction solution was stirred at 0°C for 1 hour, and then at 50 °C for 2 hours. To the reaction solution was added toluene (20 g), and the mixture was added dropwise to an ice-cooled, 5 wt% aqueous sodium hydrogen carbonate solution (38 g). The toluene layer was separated, and dried over anhydrous magnesium sulfate. Anhydrous magnesium sulfate was separated by filtration, and then the filtrate was concentrated under reduced pressure to obtain a target compound.
Amount 1.63 g
Yield 84%
¹H-NMR (toluene-d8, 400 MHz) was consistent with that in Example 27.
The stereoselectivity in the reaction was determined in the same method as in Example 27.
S-CTFAP:R-CTFAP=96:4

### [Example 31]

### Preparation of S-CHP

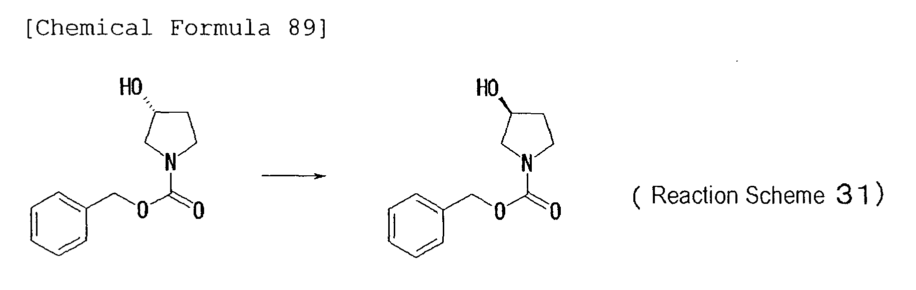

Sodium trifluoroacetate (2.04 g) was suspended in toluene (10 g), and PPDA (3.35 g) was added dropwise thereto at 0°C. The mixture was stirred at 0°C for 1 hour, and then R-CHP (1.11 g) was added thereto. The reaction solution was stirred at 0°C for 1 hour, and then at 50°C for 2 hours. To the reaction solution was added toluene (20 g), and the mixture was kept at 15°C or lower, and added dropwise to an ice-cooled, 5 wt% aqueous sodium hydrogen carbonate solution (30 g). The reaction solution was stirred at room temperature for 30 min, and then the toluene layer was separated. The toluene layer was washed three times with water (20 g), and then concentrated under reduced pressure to obtain a residue as a pale brown solid containing a target compound.
Amount 0.94 g
Yield 85%
The stereoselectivity in this reaction was determined from the peak area ratio of S-CHP and R-CHP under an HPLC analysis condition-6.
Stereoisomer ratio S-CHP:R-CHP=96:4
¹H-NMR (toluene-d8, 400 MHz) was consistent with that in Example 29.

### [Example 32]

### Preparation of S-CTFAP

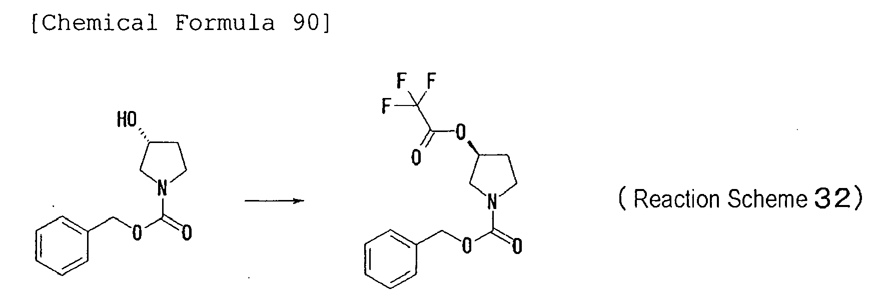

Sodium trifluoroacetate (2.04 g) was suspended in toluene (10 g), and BDDF (2.07 g) was added dropwise thereto at 0°C. The mixture was stirred at 0°C for 1 hour, and then R-CHP (1.11 g) was added thereto. The reaction solution was stirred at 0°C for 1 hour, and then at 50 °C for 2 hours. To the reaction solution was added toluene (20 g), and the mixture was added dropwise to an ice-cooled, 5 wt% aqueous sodium hydrogen carbonate solution (38 g). The toluene layer was separated, and dried over anhydrous magnesium sulfate. Anhydrous magnesium sulfate was separated by filtration, and then the filtrate was concentrated under reduced pressure to obtain a target compound.
Amount 1.55 g
Yield 80%
¹H-NMR (toluene-d8, 400 MHz) was consistent with that in Example 27.
The stereoselectivity in the reaction was determined in the same method as in Example 27.
S-CTFAP:R-CTFAP=94:6

### [Example 33]

### Preparation of S-CHP

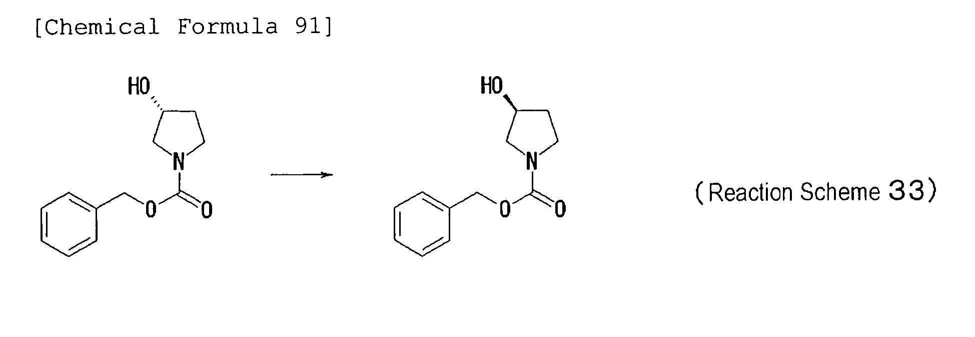

Sodium trifluoroacetate (2.04 g) was suspended in toluene (10 g), and BDDF (2.07 g) was added dropwise thereto at 0°C. The mixture was stirred at 0°C for 1 hour, and then R-CHP (1.11 g) was added thereto. The reaction solution was stirred at 0°C for 1 hour, and then at 50°C for 2 hours. To the reaction solution was added toluene (20 g), and the mixture was kept at 15°C or lower, and added dropwise to an ice-cooled, 5 wt% aqueous sodium hydrogen carbonate solution (30 g). The reaction solution was stirred at room temperature for 30 min, and then the toluene layer was separated. The toluene layer was washed three times with water (20 g), and then concentrated under reduced pressure to obtain a residue as a pale brown solid containing a target compound.
Amount 0.87 g
Yield 78%
The stereoselectivity in this reaction was determined from the peak area ratio of S-CHP and R-CHP under an HPLC analysis condition-6.
Stereoisomer ratio S-CHP:R-CHP=94:6
¹H-NMR (toluene-d8, 400 MHz) was consistent with that in Example 29.

### [Reference Example 1] Preparation of cis-TBCTS

A comparative sample of cis-TBCTS was obtained by using cis-4-tert-butylcyclohexanol, p-toluenesulfonyl chloride, and pyridine in accordance with a known process as described in [Non-Patent Document 1].

### [Reference Example 2] Preparation of trans-TBCTS

A comparative sample of trans-TBCTS was obtained by using trans-4-tert-butylcyclohexanol, p-toluenesulfonyl chloride, and pyridine in accordance with a known process as described in [Non-Patent Document 1].

### [Reference Example 3] Preparation of cis-MSBPM

A comparative sample of cis-MSBPM was obtained by using cis-N-tert-butoxycarbonyl-4-hydroxy-L-proline methylester, methanesulfonyl chloride, and pyridine in accordance with a known process as described in [Non-Patent Document 1].

### [Reference Example 4] Preparation of trans-MSBPM

A comparative sample of trans-MSBPM was obtained by using trans-N-tert-butoxycarbonyl-4-hydroxy-L-proline methylester, methanesulfonyl chloride, and pyridine in accordance with a known process as described in [Non-Patent Document 1].

### [Reference Example 5] Preparation of cis-HSBPM

A comparative sample of cis-HSBPM was obtained by using cis-N-tert-butoxycarbonyl-4-hydroxy-L-proline methylester, hexanesulfonyl chloride, and pyridine in accordance with a known process as described in [Non-Patent Document 1].

### [Reference Example 6] Preparation of trans-HSBPM

A comparative sample of trans-HSBPM was obtained by using trans-N-tert-butoxycarbonyl-4-hydroxy-L-proline methylester, methanesulfonyl chloride, and pyridine in accordance with a known process as described in [Non-Patent Document 1].

### [Reference Example 7] Preparation of cis-TSBPM

A comparative sample of cis-TSBPM was obtained by using cis-N-tert-butoxycarbonyl-4-hydroxy-L-proline methylester, p-toluenesulfonyl chloride, and pyridine in accordance with a known process as described in [Non-Patent Document 1].

### [Reference Example 8] Preparation of trans-TSBPM

A comparative sample of trans-TSBPM was obtained by using trans-N-tert-butoxycarbonyl-4-hydroxy-L-proline methylester, p-toluenesulfonyl chloride, and pyridine in accordance with a known process as described in [Non-Patent Document 1].

### [Reference Example 9] Preparation of S-BMSP

A comparative sample of S-BMSP was obtained by using 3S-N-tert-butoxycarbonyl-3-hydroxypyrrolidine, methanesulfonyl chloride, and pyridine in accordance with a known process as described in [Non-Patent Document 1].

### [Reference Example 10] Preparation of S-BHSP

A comparative sample of S-BHSP was obtained by using 3S-N-tert-butoxycarbonyl-3-hydroxypyrrolidine, hexanesulfonyl chloride, and pyridine in accordance with a known process as described in [Non-Patent Document 1].

### [Reference Example 11] Preparation of S-BTSP

A comparative sample of S-BTSP was obtained by using 3S-N-tert-butoxycarbonyl-3-hydroxypyrrolidine, p-toluenesulfonyl chloride, and pyridine in accordance with a known process as described in [Non-Patent Document 1].

### [Reference Example 12] Derivation to R-BPP from S-BMSP

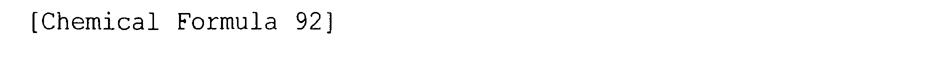

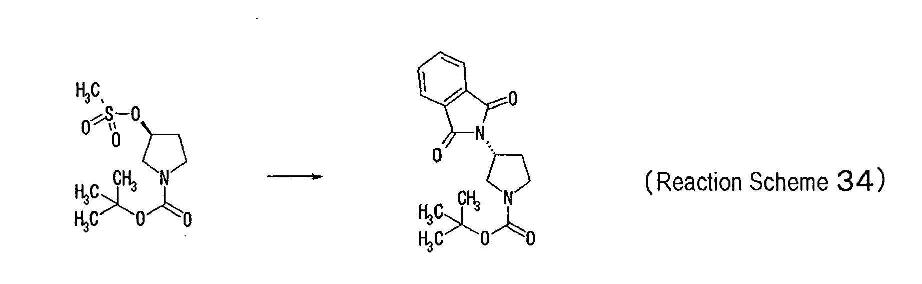

S-BMSP (2.65 g) was dissolved in N,N-dimethyl formamide (30 g), and phthalimidepotassium (2.22 g) was added thereto. The reaction solution was heated to 85°C, and stirred for 5 hours. The obtained reaction solution was used as a sample for measurement of the optical purity. Further, a part of the sample was isolated to confirm its structure.
¹H-NMR (CDCl₃, 270 MHz) δ 7.89-7.83 (m, 2H), 7.78-7.72 (m, 2H), 4.86 (dddd, 1H, J=8.0 Hz), 3.74-3.62 (m, 3H), 3.46-3.36 (m, 1H), 2.68-2.56 (m, 1H), 2.17-2.05 (m, 1H), 1.47 (s, 9H)
HPLC analysis condition 5
Chiral column used DAICEL CHIRALCEL OJ
Eluent hexane/2-propanol=90/10
Flow rate 1 ml/min
Detection wavelength UV 210 nm
Column temperature 25°C

### INDUSTRIAL AVAILABILTY

According to the present invention, a sulfonic acid ester using alcohol as a raw material can be prepared in a simple manner and with a high yield. Further, a sulfonic acid ester, a carboxylic acid ester and alcohol can be derived by involving inversion of steric configuration with respect to alcohol serving as a raw material. In addition, in the present process, the by-products derived from the reaction reagent are easily removed, and there is no risk of explosion, thus providing high stability. Therefore, the invention provides a very efficient novel preparation process from an industrial viewpoint, for functional products including raw materials for pharmaceuticals, agrochemicals, cosmetics and the like, and it has high utility.

## Claims

1. A process for producing an ester or alcohol having an inverted steric configuration with respect to alcohol serving as a raw material, the process comprising reacting an optically active alcohol or a geometrically isomeric alcohol with a compound represented by the general formula (1): 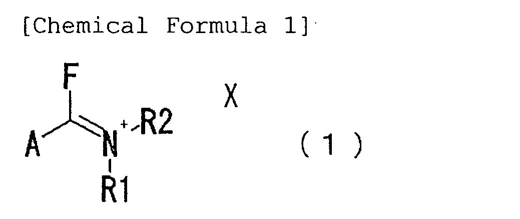 [wherein R1 and R2 each independently represents a substituted or unsubstituted alkyl group, a substituted or unsubstituted aryl group, or a substituted or unsubstituted hetero ring, and may be the same as or different from each other. Further, R1 and R2 may be bonded to each other to form a ring containing a nitrogen atom, or a nitrogen atom and other heteroatom. A represents a hydrogen atom, a substituent represented by the general formula (2): 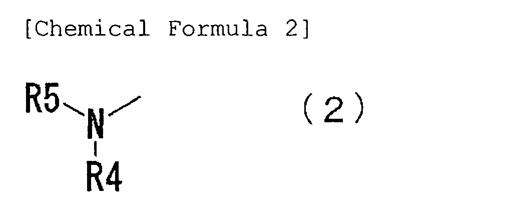 (wherein R4 and R5 each independently represents a substituted or unsubstituted alkyl group, a substituted or unsubstituted aryl group, or a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.), or a substituent represented by the general formula (3): 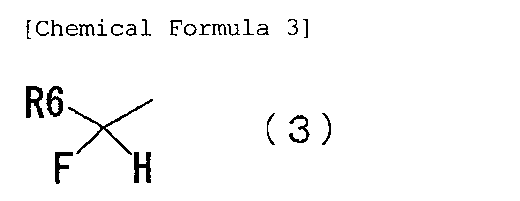 (wherein R6 represents a fluorine atom, or an alkyl group that is partially or fully substituted with fluorine atom(s)). X represents a compound represented by the general formula (4): 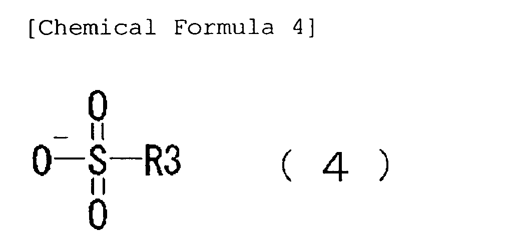 (wherein R3 represents a substituted or unsubstituted alkyl group, a substituted or unsubstituted aryl group, or a substituted or unsubstituted hetero ring), or by the general formula (5): 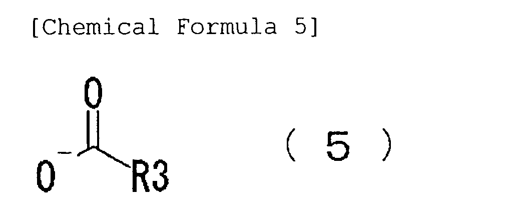 (wherein R3 represents a substituted or unsubstituted alkyl group, a substituted or unsubstituted aryl group, or a substituted or unsubstituted hetero ring)].

2. The process for producing an ester or alcohol as set forth in claim 1, wherein in the compound represented by the general formula (1), X is represented by the general formula (4), and the process comprises obtaining a sulfonic acid ester.

3. The process for producing an ester or alcohol as set forth in claim 1, wherein the compound represented by the general formula (1) is represented by the general formula (6): 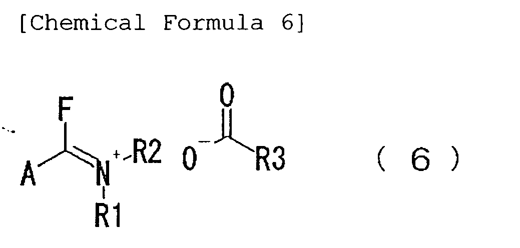 (wherein R1, R2, R3 and A each have the same meanings as defined in the general formula (1)), and the process comprises obtaining a carboxylic acid ester or alcohol.

4. The process for producing an ester or alcohol as set forth in claim 3, wherein the compound represented by the general formula (6) is represented by the general formula (7): 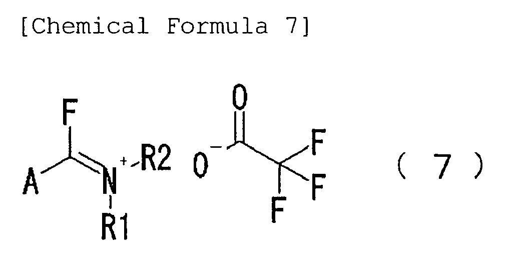 (wherein R1, R2 and A each have the same meanings as defined in the general formula (1)), and the process comprises obtaining a carboxylic acid ester or alcohol.

5. The process for producing an ester or alcohol as set forth in claim 2, wherein the optically active alcohol or the geometrically isomeric alcohol is an optically active secondary alcohol, and the process comprises obtaining a sulfonic acid ester.

6. The process for producing an ester or alcohol as set forth in claim 3 or 4, wherein the optically active alcohol or the geometrically isomeric alcohol is an optically active secondary alcohol, and the process comprises obtaining a carboxylic acid ester or alcohol.

7. The process for producing an ester or alcohol as set forth in claim 5, wherein the optically active secondary alcohol is represented by the general formula (8): 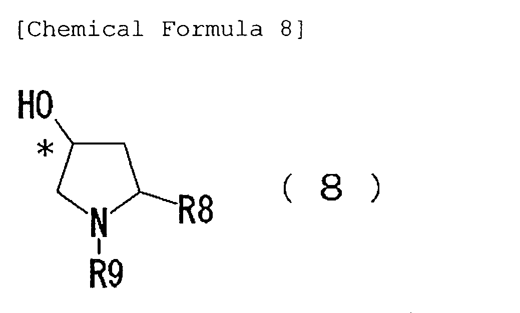 (wherein R8 represents a hydrogen atom, a carboxylic acid, a carboxylic acid ester, or a carboxylic acid amide. R9 represents a substituted or unsubstituted alkyl group, a substituted or unsubstituted aryl group, a substituted or unsubstituted alkoxycarbonyl group, a substituted or unsubstituted aryloxycarbonyl group, a substituted or unsubstituted alkylcarbonyl group, or a substituted or unsubstituted arylcarbonyl group. The symbol * represents that the hydroxyl group is optically active, indicating a compound in either an R- or an S-form.), and the process comprises obtaining a sulfonic acid ester.

8. The process for producing an ester or alcohol as set forth in claim 6, wherein the optically active secondary alcohol is represented by the general formula (8): 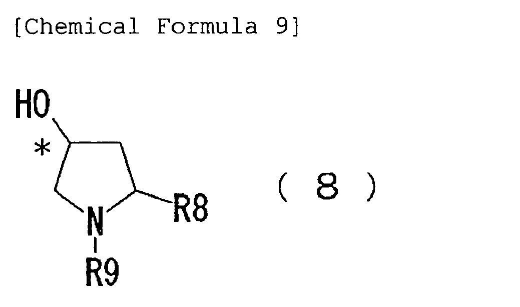 (wherein R8 represents a hydrogen atom, a carboxylic acid, a carboxylic acid ester, or a carboxylic acid amide. R9 represents a substituted or unsubstituted alkyl group, a substituted or unsubstituted aryl group, a substituted or unsubstituted alkoxycarbonyl group, a substituted or unsubstituted aryloxycarbonyl group, a substituted or unsubstituted alkylcarbonyl group, or a substituted or unsubstituted arylcarbonyl group. The symbol * represents that the hydroxyl group is optically active, indicating a compound in either an R- or an S-form.), and the process comprises obtaining a carboxylic acid ester or alcohol.

9. A compound represented by the general formula (9): 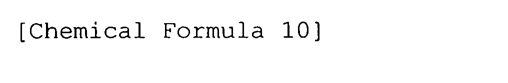 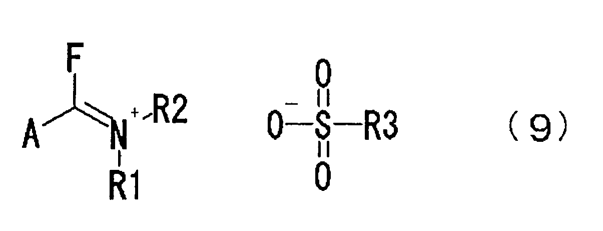 [wherein R1, R2, and R3 each independently represents a substituted or unsubstituted alkyl group, a substituted or unsubstituted aryl group, or a substituted or unsubstituted hetero ring, and may be the same as or different from each other. Further, R1 and R2 may be bonded to each other to form a ring containing a nitrogen atom, or a nitrogen atom and other heteroatom. A represents a hydrogen atom, a substituent represented by the general formula (2) 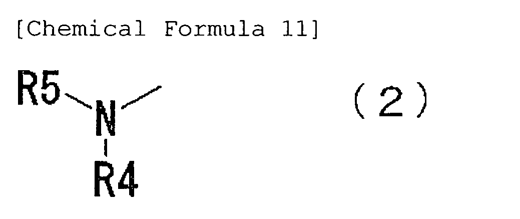 (wherein R4 and R5 each independently represents a substituted or unsubstituted alkyl group, a substituted or unsubstituted aryl group, a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.), or a substituent represented by the general formula (3) 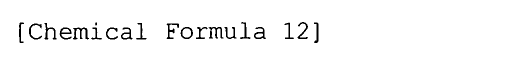 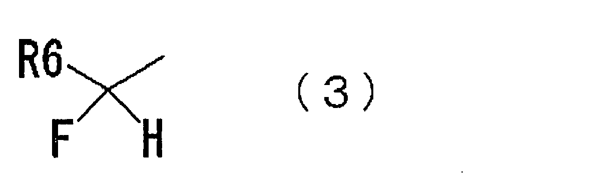 (wherein R6 represents a fluorine atom, or an alkyl group that is partially or fully substituted with fluorine atom(s))].

10. The compound as set forth in claim 9, wherein the compound represented by the general formula (9) is represented by the general formula (10): 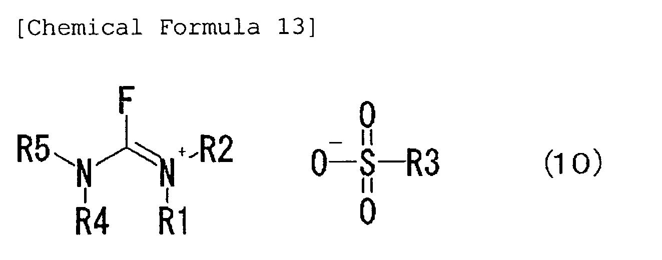 (wherein R1 to R5 each have the same meanings as defined in the general formula (9)).

11. The compound as set forth in claim 9, wherein the compound represented by the general formula (9) is represented by the general formula (11): 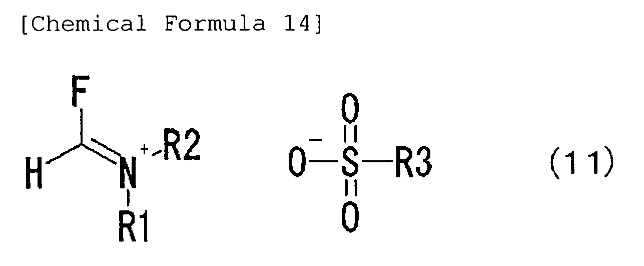 (wherein R1 to R3 each have the same meanings as defined in the general formula (9)).

12. The compound as set forth in claim 9, wherein the compound represented by the general formula (9) is represented by the general formula (12): 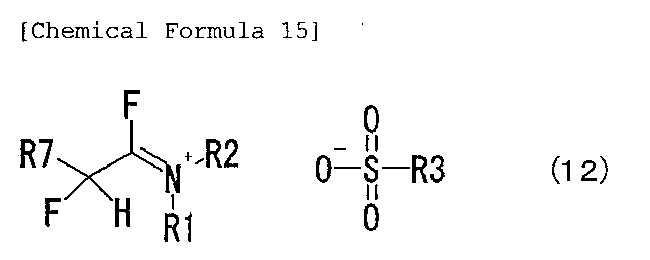 (wherein R1 to R3 each have the same meanings as defined in the general formula (9). R7 represents a fluorine atom, or an alkyl group having 1 to 6 carbon atoms, that is fully substituted with fluorine atoms).

13. The compound as set forth in claim 10, wherein in the compound represented by the general formula (10), R1, R2, R4, and R5 are each independently an alkyl group having 1 to 6 carbon atoms.

14. The compound as set forth in claim 10, wherein the compound represented by the general formula (10) is represented by the general formula (13): 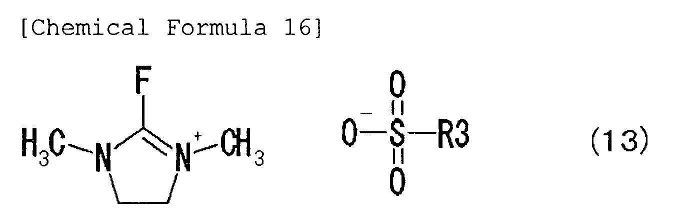 (wherein R3 has the same meaning as defined in the general formula (9)).

15. A process for producing a compound represented by the general formula (9): 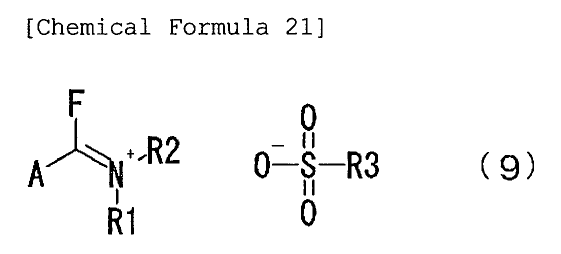 [wherein R1, R2, R3, and A each have the same meanings as defined below],
the process comprising reacting a compound represented by the general formula (14): 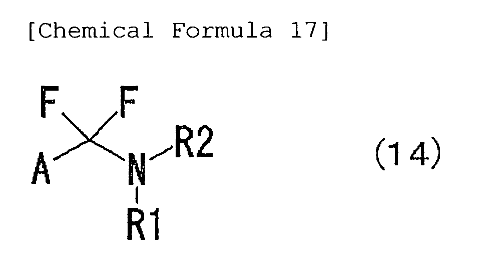 [wherein R1 and R2 each independently represents a substituted or unsubstituted alkyl group, a substituted or unsubstituted aryl group, or a substituted or unsubstituted hetero ring, and may be the same as or different from each other, while R1 and R2 may be bonded to each other to form a ring containing a nitrogen atom, or a nitrogen atom and other heteroatom; A represents a hydrogen atom, a substituent represented by the general formula (2) 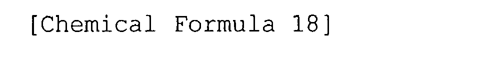 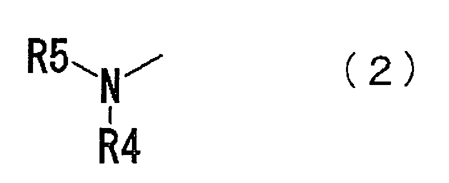 (wherein R4 and R5 each independently represents a substituted or unsubstituted alkyl group, a substituted or unsubstituted aryl group, or a substituted or unsubstituted hetero ring, and may be the same as or different from each other, where R4 and R5 may be bonded to each other to form a ring containing a nitrogen atom, or a nitrogen atom and other heteroatom; alternatively, R1 and R4 may be bonded to each other to form a ring containing a nitrogen atom.), or a substituent represented by the general formula (3): 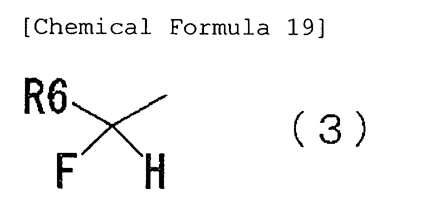 (wherein R6 represents a fluorine atom, or an alkyl group that is partially or fully substituted with fluorine atom(s)); any one of the fluorine atoms on the carbon atoms bonded with the nitrogen atoms may be anions to form ion-pairs] with a compound represented by the general formula (15): 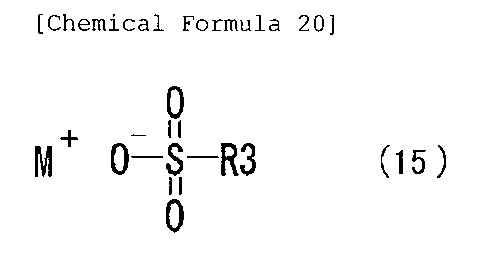 (wherein R3 represents a substituted or unsubstituted alkyl group, a substituted or unsubstituted aryl group, or a substituted or unsubstituted hetero ring; M⁺ represents an alkali metal ion or an alkaline earth metal ion).

16. The process as set forth in claim 15, wherein the compound represented by the general formula (14) is represented by the general formula (16): 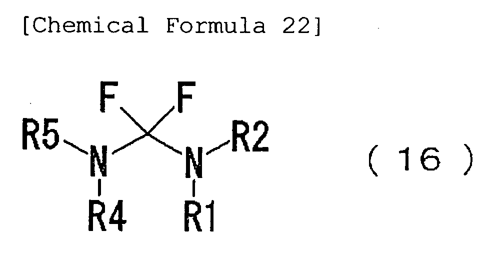 (wherein R1, R2, R4, and R5 each have the same meanings as defined in the general formula (14). Further, any one of the fluorine atoms on the carbon atoms bonded with the nitrogen atoms may be anions to form ion-pairs.).

17. The process as set forth in claim 15, wherein the compound represented by the general formula (14) is represented by the general formula (17): 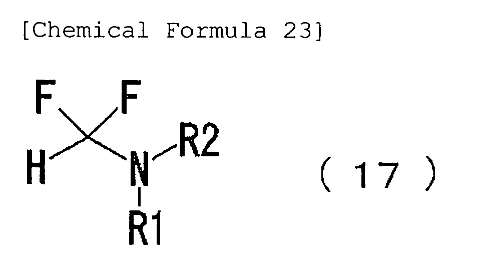 (wherein R1 and R2 each have the same meanings as defined in the general formula (14). Further, any one of the fluorine atoms on the carbon atoms bonded with the nitrogen atoms may be anions to form ion-pairs.).

18. The process as set forth in claim 15, wherein the compound represented by the general formula (14) is represented by the general formula (18): 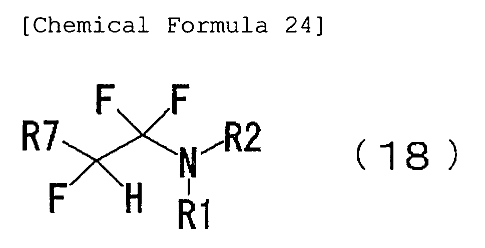 (wherein R1 and R2 each have the same meanings as defined in the general formula (14). R7 represents a fluorine atom, or an alkyl group having 1 to 6 carbon atoms, that is fully substituted with fluorine atoms. Further, any one of the fluorine atoms on the carbon atoms bonded with the nitrogen atoms may be anions to form ion-pairs. Alternatively, the hydrogen fluoride may leave to make an olefinic compound.).

19. The process as set forth in claim 15, wherein the compound represented by the general formula (14) is the compound in which R1, R2, R4, and R5 are each an alkyl group having 1 to 6 carbon atoms.

20. The process as set forth in claim 15, wherein the compound represented by the general formula (14) is represented by the formula (19):

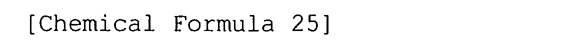

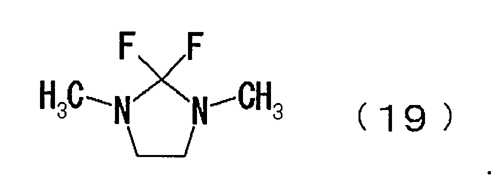

21. A process for producing a sulfonic acid ester, comprising reacting alcohol with a compound represented by the general formula (9) : 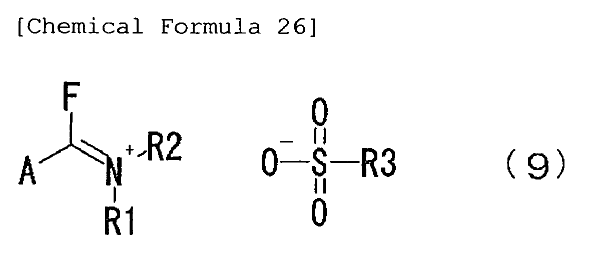 [wherein R1, R2, and R3 each independently represents a substituted or unsubstituted alkyl group, a substituted or unsubstituted aryl group, a substituted or unsubstituted hetero ring, and may be the same as or different from each other. Further, R1 and R2 may be bonded to each other to form a ring containing a nitrogen atom, or a nitrogen atom and other heteroatom. A represents a hydrogen atom, a substituent represented by the general formula (2) 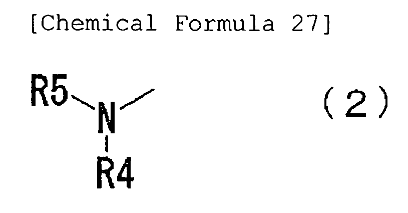 (wherein R4 and R5 each independently represents a substituted or unsubstituted alkyl group, a substituted or unsubstituted aryl group, or a substituted or unsubstituted hetero ring, and may be the same as or different from each other. Further, R4 and R5 may be bonded to each other to form a ring containing a nitrogen atom, or a nitrogen atom and other heteroatom. Alternatively, R1 and R4 may be bonded to each other to form a ring containing a nitrogen atom.), or a substituent represented by the general formula (3) 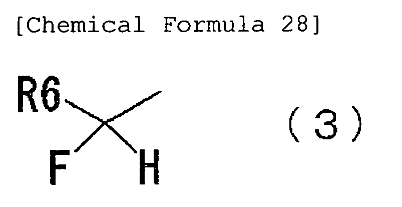 (wherein R6 represents a fluorine atom, or an alkyl group that is partially or fully substituted with fluorine atom(s).).
